# EUROPEAN PATENT APPLICATION

(11) **EP 3 970 644 A1**
(43) Date of publication of application: **23.03.2022**
(21) Application number: 20798107.7
(22) Date of filing: 01.05.2020
(51) Int. Cl.: A61B 17/34

(54) **SUTURE LINE RELEASE MECHANISM, PUNCTURING CORE COMPONENT, PUNCTURE OUTFIT, AND USE METHOD THEREOF**

(30) Priority: 02.05.2019 CN 201910367365
(71) Applicant: Fulbright Medical Inc., Wuxi, Jiangsu 214437 (CN)
(72) Inventor: SUN, Baofeng, Wuxi, Jiangsu 214437 (CN); HUANG, Ye, Wuxi, Jiangsu 214437 (CN)
(74) Representative: Novagraaf Technologies
(86) International application number: PCT/CN2020/088552
(87) International publication number: WO 2020/221370

(57) **Abstract**

The present disclosure provides a suture line release mechanism, the puncture core assembly, a puncture device, and a use method of the puncture device. The suture line release mechanism includes a release execution component; the release execution component includes a fixing member and a puncturing tip; the puncturing tip is provided with a receiving part; the release execution component has an initial state and a release state; in the initial state, a part of the fixing member is received in the receiving part, and a closed suture line receiving space is formed between the puncturing tip and the part of the fixing member; in the release state, the suture line receiving space is exposed.

## Description

### Technical Field

The present disclosure relates to a suture line release mechanism for a puncture core assembly, the puncture core assembly, a suturable puncture device with the puncture core assembly, and a use method of the suturable puncture device, and belongs to a field of medical equipment.

### Background

In minimally invasive surgery such as abdominal surgery and thoracic surgery, a puncture device may establish an access channel in a cavity wall of a human body, so that a stapler or other surgical instruments (such as an endoscope) may enter a body cavity, and provide a gas access channel for inspection or surgical operation.

The puncture device includes a puncture core assembly and a cannula component. During an operation, a doctor usually cuts a small incision on the human tissue of a patient first, then aligns a puncturing tip of the puncture core assembly with the cut small incision, performs left and right rotation in a reciprocating manner, and downwardly moves the puncture device at the same time, so that the puncture core assembly guides the cannula component to penetrate through the cortex of the human tissue of the patient, then the doctor pulls out the puncture core assembly, and the stapler or other surgical instruments may enter and exit a body cavity of the patient through the cannula component for operation. An existing puncture core assembly only plays the role of puncturing, and is discarded after guiding the cannula component from an incision of a human abdomen into the body of the patient.

At an end of the operation, the cannula component is taken out from a puncture opening, and the puncture opening is sutured. Because the puncture opening of minimally invasive surgery is small and deep, especially the human tissue of an obese patient is relatively thick, if the suture is improper, the patient is prone to complications such as puncture hole hernia after operation. A special suture instrument may be used to suture the puncture opening to reduce the above complications, but this requires additional surgical instruments, relatively high suture cost, a large number of surgical instruments and inconvenient operation. Moreover, a suture device specially suturing the puncture opening is complex in structure and inconvenient to use. Therefore, the puncture device with a suture function may be used.

The puncture device with a suture function needs to put a suture line in the puncture core assembly to make the suture line follow the puncture core assembly to enter the puncture opening. Chinese patent application No. CN201811283165.9 discloses a puncture device with a suture function, which includes a puncture device and a suture component inserted into a puncture cannula. The suture component includes an outer pipe and a push rod. The head of the outer pipe is provided with a gear with a hook, and the head of the hook of the gear is provided with a needle. A side hole of the needle is provided with a suture line of corresponding specification. The head of the push rod is provided with a rack matched with the gear with the hook. The rack is cooperated with the gear with the hook to realize an arc rotation of the needle. The push rod is pressed down, the hook is opened and then closed, the needle passes through a suture part and is inserted into a symmetrical hole of the head of the cannula of the puncture device, then the suture component is opened, the needle is separated from the hook, and the head of the suture line is retained in the side wall of the puncture device.

However, the puncture device of Chinese patent application No. CN201811283165.9 disperses the suture line outside the side wall of the puncture device, which is not conducive to receive the suture line.

### Summary

In order to solve the above problems, the present disclosure provides a suture line release mechanism for a puncture core assembly, the puncture core assembly and a puncture device with the puncture core assembly.

A suture line release mechanism for a puncture core assembly is provided. The suture line release mechanism includes a release execution component, the release execution component includes a fixing member and a puncturing tip, the puncturing tip is provided with a receiving part, the release execution component has an initial state and a release state, in the initial state, a part of the fixing member is received in the receiving part, a closed suture line receiving space is formed between the puncturing tip and the part of the fixing member, and in the release state, the suture line receiving space is exposed.

Further, the puncturing tip moves along an axial direction of the puncture core assembly, so that the part of the fixing member is received in the receiving part or at least part of the part of the fixing member is removed from the receiving part, so that the release execution component is switched between the initial state and the release state.

Further, the release execution component also includes a lock cylinder fixed relative to the puncturing tip, the suture line release mechanism also includes a release transmission component, the release transmission component drives the lock cylinder to move along the axial direction, and an axial movement of the lock cylinder drives the puncturing tip to move along the axial direction.

Further, the lock cylinder includes a hook, the fixing member includes a first holding part and a second holding part, and the first holding part and the second holding part are disposed at intervals along the axial direction. The hook is cooperated with the first holding part to keep the release execution component in the initial state. The hook is cooperated with the second holding part to keep the release execution component in the release state.

Further, an axial guide mechanism is disposed between the lock cylinder and the fixing member.

Further, the release transmission component includes a transmission rod, and the transmission rod butts against the lock cylinder to push the lock cylinder to move axially.

Further, the lock cylinder is provided with an opening groove, the opening groove extends in a direction from the near end of the lock cylinder towards the far end of the lock cylinder, a far end of the opening groove is closed to form a far end face, and a far end part of the transmission rod butts against the far end face of the opening groove to push the lock cylinder to move axially.

Further, the transmission rod separably butts against the lock cylinder along the axial direction.

Further, the fixing member is provided with a through hole that extending axially, and the lock cylinder is movably received in the through hole.

Further, a far end part of the lock cylinder located outside the through hole is fixedly connected with the puncturing tip.

A puncture core assembly is provided. The puncture core assembly includes the suture line release mechanism mentioned above.

Further, the suture line release mechanism includes a release operating component, the puncture core assembly also includes a suture mechanism, the suture mechanism includes a suture operating component, and the release operating component and the suture operating component are a same operating component.

Further, the puncture core assembly also includes a suture needle fixing mechanism, the suture needle fixing mechanism includes a suture needle fixing operating component, and the suture needle fixing operating component, the suture operating component and the release operating component are the same operating component.

Further, the same operating component includes a misoperation prevention mechanism, the misoperation prevention mechanism includes a first part and a second part, the first part includes an adjusting block and a spring, one end of the spring butts against the adjusting block, the other end of the spring butts against other adjusting blocks or other parts of the first part except the adjusting block, and the second part includes a blocking part. The blocking part is provided with a first guide inclined plane, and the adjusting block is provided with a second guide inclined plane. In an initial position, the first guide inclined plane butts against the second guide inclined plane. When a force of misoperation of the first part is not enough to overcome an elastic force of the spring, the blocking part butts against the adjusting block to prevent the first part from moving relative to the second part due to misoperation. When an operating force is increased, the force overcomes the elastic force of the spring to make the adjusting block move towards other adjusting blocks or the other parts of the first part along the guide inclined plane. When the adjusting block is separated from the blocking part, the first part is operated to move relative to the second part.

Further, the suture line release mechanism includes a suture line release transmission component, the puncture core assembly also includes a suture mechanism, the suture mechanism includes a suture transmission component, the suture line release transmission component shares a transmission rod with the suture transmission component, and the transmission rod includes a suture driving part and a suture line release driving part. The suture driving part is a toothed segment disposed on a side surface of the transmission rod, and the suture line release driving part is a far end part of the transmission rod.

A suturable puncture device includes the puncture core assembly and a cannula component mentioned above. The puncture core assembly is detachably sleeved in the cannula component.

A use method of a suturable puncture device is provided. The suturable puncture device includes a puncture core assembly and a cannula component. The puncture core assembly is detachably sleeved in the cannula component. The puncture core assembly includes a first operating component, a second operating component, a third operating component, a positioning blade, a suture piece and a suture line, and the suture piece includes a suture needle. The puncture core assembly includes a puncturing tip and a fixing member, and a suture line receiving space is formed between the puncturing tip and the fixing member. The use method includes the following steps.

step S1: operating the first operating component, so as to rotate the positioning blade outwardly, and make the positioning blade protrude out of an outer surface of the puncture core assembly.

step S2: operating the second operating component, so as to rotate the suture piece.

step S3: operating the second operating component, so as to allow the suture line received in the suture line receiving space to be released.

step S4: operating the third operating component, so as to hold the suture needle.

Further, in the step S1, an operation is rotation.

Further, the step S1 also includes: exposing a suture channel after the positioning blade rotates outwardly.

Further, in the step S2 and the step S3, an operation is pressing.

Further, the second operating component includes a pressure cover. The step S3 also includes: pressing the pressure cover to enable the pressure cover to move downwardly. The pressure cover moves downwardly to drive the puncturing tip to move downwardly, so as to expose the suture line receiving space, and a rotation of the suture needle starts synchronously with an exposure of the suture line receiving space.

Further, a step S11 is also included between the step S1 and the step S2: upwardly pulling the puncture core assembly or the suturable puncture device, so as to realize a positioning of the positioning blade.

Further, the step S4 also includes: pressing the third operating component to push a third transmission component, the third transmission component pushes a movable piece to move downwardly, and a slit of the movable piece holds the suture needle.

Further, the use method also includes the following steps.

step S5: operating the second operating component, so as to make the suture piece return.

step S6: operating the second operating component, so as to close the suture line receiving space.

step S7: operating the first operating component, so as to rotate the positioning blade inwardly.

step S8: upwardly pulling the puncture core assembly or the suturable puncture device.

Further, in the step S5 and the step S6, an operation is pulling.

Further, in the step S7, an operation is rotation.

Further, the second operating component includes a pressure cover. The step S6 also includes: pulling the pressure cover to enable the pressure cover to move upwardly, and the pressure cover moves upwardly to drive the puncturing tip to move upwardly, so as to close the suture line receiving space.

Compared with the prior art, the present disclosure has the beneficial effects that: due to the arrangement of the fixing member, in the initial state, by providing a closed suture line receiving space between the fixing member and the puncturing tip, when the puncture core assembly does not perform a suture action, a suture line is well received in the suture line receiving space to prevent the scattered suture line from affecting the normal operation of the surgery. Moreover, the space between the fixing member and the puncturing tip is used to receive the suture line without changing a shape of the puncturing tip, which will not cause an adverse impact on the operation of the puncturing tip piercing the cortex of a patient.

### Brief Description of the Drawings

Fig. 1 is a schematic structural diagram of a puncture device provided by a first embodiment of the present disclosure.
Fig. 2 is a schematic structural diagram of a puncture core assembly provided by the present disclosure.
Fig. 3 is an explosive view of Fig. 2.
Fig. 4 is a schematic structural diagram of parts required to achieve positioning when the positioning is not performed.
Fig. 5 is a connection schematic diagram of a rotating ring and a positioning piece.
Fig. 6 is a schematic structural diagram of parts required to achieve positioning after the positioning is performed.
Fig. 7 is a schematic structural diagram of parts driven by a pressure cover to move.
Fig. 8 is a schematic structural diagram of the pressure cover at an angle.
Fig. 9 is a schematic structural diagram of Fig. 8 at another angle.
Fig. 10 is a schematic structural diagram of an adjusting frame and an adjusting block.
Fig. 11 is a schematic diagram of a suture component when a needle has not extended out.
Fig. 12 is a schematic structural diagram of a receiving piece.
Fig. 13 is a schematic structural diagram of the puncture core assembly when a needle extending action, a needle receiving action and a suture line releasing action are completed.
Fig. 14 is a sectional view of the puncture core assembly in the state of Fig. 12.
Fig. 15 is a schematic structural diagram of parts required to achieve the needle receiving action and a needle fixing action.
Fig. 16 is a schematic structural diagram of driving a pressure disc in a second position.
Fig. 17 is a schematic structural diagram of a needle fixing component when the needle fixing action is completed.
Fig. 18 is a schematic structural diagram of the puncture core assembly after the suture component is reset.
Fig. 19 is a three-dimensional exploded view of a puncture core assembly provided by a second embodiment of the present disclosure.
Fig. 20 is a three-dimensional exploded view of a part of parts of the puncture core assembly illustrated in Fig. 19 in another angle.
Fig. 21 is a part sectioned view of the puncture core assembly illustrated in Fig. 19, at this time, a release execution component is in an initial state.
Fig. 22 is another part sectioned view of the puncture core assembly illustrated in Fig. 19, at this time, the release execution component is in a release state.

Reference numerals:
1-operating component,
11-turntable, 111-lower housing, 112-shift arm, 113-blocking sheet, 113a-first guide inclined plane,
12-pressure cover, 121-pressing disc, 122-notch, 122a-slit, 1221-rib plate group, 123-circumferential wall, 124-pushing member, 1241-pushing member plate,
2-butting disc, 21-central through hole,
3-rod wall pipe, 31-window, 32-deformation sheet,
4-transmission component,
41-first transmission component, 411-first transmission pipe, 412-transmission arm, 413-rotating ring,
42-second transmission component, 421-adjusting frame, 4212-guide groove, 422-adjusting block, 422a-second guide inclined plane, 4221-guide protruding block, 4222-anti-shaking protruding strip, 423-elastic piece, 424-transmission rod, 424a-near end, 424b-far end, 425-rack, 425a-upper toothed segment, 425b-lower toothed segment,
43-third transmission component, 431-upper transmission ring, 432-connecting rod, 433-third transmission pipe, 434-lower transmission ring, 435-booster arm,
5-supporting component, 5'-upper half part, 5"-lower half part, 51-first supporting piece, 511-avoidance space, 52-second supporting piece, 53-suture channel, 54-avoidance groove, 541,542-groove wall, 55-receiving hole,
6-execution component,
61-positioning component, 611-movement aiding arm, 6111-waist-shaped groove, 612-pivot shaft, 613-positioning blade, 614-protruding part,
62-suture component, 621,622-suture piece, 623-gear, 624-rotating shaft, 625-first suture arm, 626-second suture arm, 627-suture needle,
63-receiving component, 631,632-receiving piece, 6311-receiving part, 6312-holding part, 6313-receiving sheet, 6314-blocking arm,
64-needle fixing component, 641,642-movable piece, 641a-limiting slit,
7-puncturing tip, 71 -first wall housing, 72-second wall housing, 711,721-toothed piece, 7111-first limiting protruding block, 712-first supporting shaft, 722-second supporting shaft, 73-protrusion,
8-insertion block component, 81-button, 82-hook, 83-spring,
9-cover body, 91-circular hole, 92-circumferential through hole.

### Detailed Description of the Embodiments

The embodiments of the present disclosure will be described below in detail and examples of the embodiments are shown in the drawings. In the description of the present disclosure, "several" means at least one, unless otherwise expressly and specifically defined.

The embodiments described below with reference to the drawings are exemplary and intended to explain the present disclosure and should not be understood as limits to the present disclosure.

In order to simplify the description, in the embodiment of the present disclosure, the ends of all parts close to a doctor are set as "near ends" or "above", and the ends away from the doctor, that is, the ends close to the body of a patient are set as "far ends" or "below". Connection includes both direct connection and indirect connection. Connection includes fixed connection, movable connection, separable connection and the like, unless otherwise definitely limited.

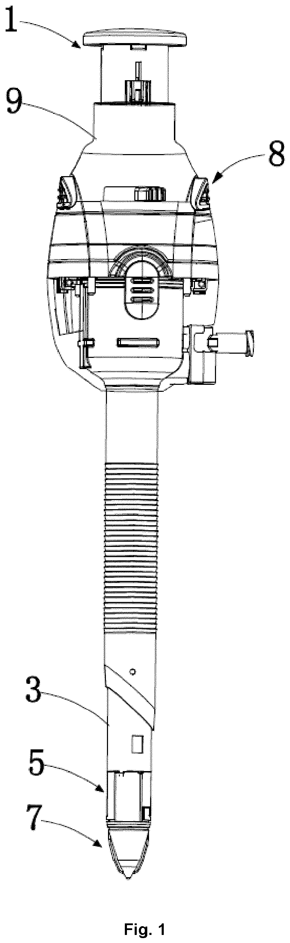

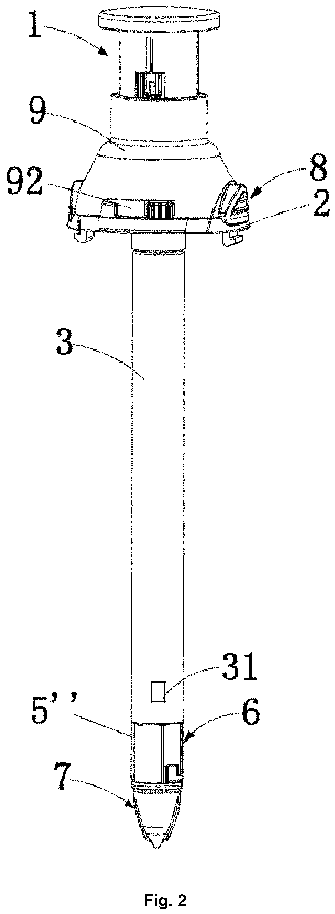

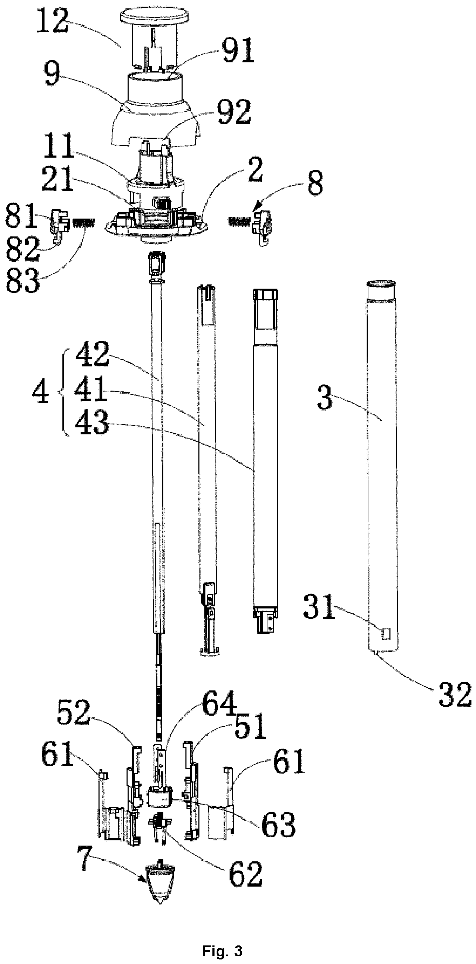

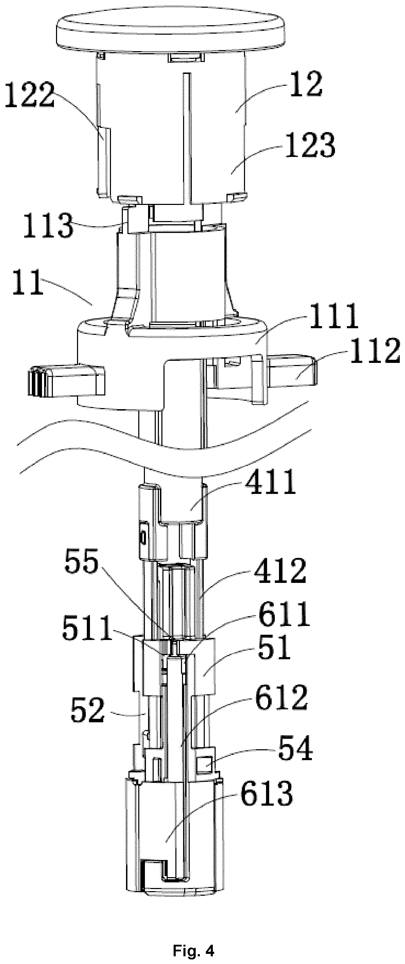

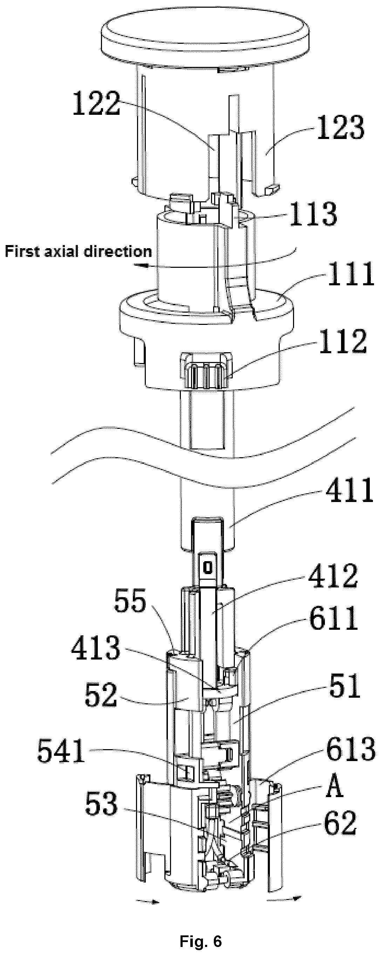

Referring to Fig. 1, the present disclosure provides a puncture device, which includes a cannula component (not labeled) and a puncture core assembly partly disposed in the cannula component in a sleeving manner. Referring to Fig. 2 and Fig. 3, the puncture core assembly includes an operating component 1, a butting disc 2, a rod wall pipe 3, a transmission component 4, a supporting component 5, an execution component 6, a puncturing tip 8, an insertion block component 8 and a cover body 9. The cover body 9 covers the upper surface of the butting disc 2 to prevent foreign matters from falling into the butting disc 2 and affecting the realization of the function of the puncture core assembly. A cylinder 91 is disposed in the center of the cover body 9, and the cylinder 91 is sleeved on the outside of the operating component 1. The part of the operating component 1 exposed to the cylinder 91 is manipulated by a doctor. Inside the cylinder 91, the operating component 1 is connected with the transmission component 4. The center of the butting disc 2 is provided with a central through hole 21 coaxial with the cylinder 91, and the transmission component 4 is disposed in the central through hole 21 in a penetrating manner. The supporting component 5 is below the transmission component 4, and the supporting component 5 is used to support and protect the execution component 6. The supporting component 5 includes two supporting pieces with the same structure, namely the first supporting piece 51 and the second supporting piece 52. The rod wall pipe 3 is sleeved on the outside of the transmission component 4, the near end of the rod wall pipe 3 is connected with the lower surface of the butting disc 2, and the rod wall pipe 3 is coaxial with the central through hole 21. The far end of the rod wall pipe 3 surrounds an upper half part 5' of the supporting component 5 and is fixedly connected with the upper half part 5'. A lower half part 5" of the supporting component 5 is exposed between the rod wall pipe 3 and the puncturing tip 7, and the outer surface of the lower half part 5" is flush with the outer surface of the rod wall pipe 3, so that the puncture core assembly enters and exits a puncture opening without hindrance. The outer surface of the rod wall pipe 3 is the outer surface of the puncture core assembly. The end face of the far end of the rod wall pipe 3 extends downwardly to form two symmetrical deformation sheets 32. The junction of the upper half part 5' and the lower half part 5" is provided with an avoidance groove 54. As illustrated in Fig. 4 and Fig. 6, a groove wall 541 of the avoidance groove 54 is located between the end face of the far end of the deformation sheet 32 and the end face of the far end of the rod wall pipe 3. When the rod wall pipe 3 is sleeved on the outside of the upper half part 5', the avoidance groove 54 provides a space for the deformation sheet 32 to bend. A deformation tool is used to bend the deformation sheet 32 inwardly to form a hook (not shown in the figures) and make the hook butted against the groove wall 541. In this way, the groove wall 541 prevents the hook from moving axially, thereby preventing the rod wall pipe 3 from moving axially. Moreover, the rod wall pipe 3 is provided with at least one first pin hole (not shown in the figures), and the upper half part of the supporting component 5 is provided with at least one second pin hole (not shown in the figures). When the rod wall pipe 3 is sleeved on the outside of the upper half part of the supporting component 5, the first pin hole and the second pin hole are made to be coaxial, a fixed pin (not shown in the figures) is used to be sequentially inserted into the first pin hole and the second pin hole which are coaxial, thus, the rod wall pipe 3 is fixed with the upper half part of the supporting component 5, and then the rod wall pipe 3 is connected with the supporting component 5. The cannula component is sleeved on the outside of the rod wall pipe 3, and the near end of the cannula component is separably connected with the lower surface of the butting disc 2. The insertion block component 8 is used to realize the separable connection. The insertion block component 8 includes two symmetrical insertion block pieces (not labeled), and each insertion block piece includes a button 81, a hook 82 and a spring 83. The button 81 is movably connected to the upper part of the butting disc 2, the hook 82 is fixedly connected to the button 81 and extends from the button 81 to the lower part of the butting disc 2, the spring 83 is located in the cover body 9, one end of the spring 83 butts against the button 81, and the other end of the spring 83 butts against the vertical part of the butting disc 2. The hook 82 can be clamped with the cannula component. The button 81 and the spring 83 are used to move the hook 82 to separate the hook 82 from the cannula component, so as to separate the butting disc 2 from the near end of the cannula component. The insertion block component 8 belongs to the prior art and will not be elaborated. In the present disclosure, based on the positional relationship shown in Fig. 2, the end where the puncturing tip 7 is located in the puncture core assembly is called the far end or below, the end where the operating component 1 is located in the puncture core assembly is called the near end or above, the side close to the central axis of the rod wall pipe 3 is called the inner side, the side away from the central axis of the rod wall pipe 3 is called the outer side, the radius direction of the rod wall pipe 3 is called the radial direction, the direction of the central axis of the rod wall pipe 3 is called the axial direction, the direction perpendicular to the axial direction is called the transverse direction, the transverse direction includes the radial direction, the direction perpendicular to the central axis of the rod wall pipe 3 and parallel to the circumference of the rod wall pipe 3 is defined as the circumferential direction, and the surface parallel to the circumferential direction is defined as the circumferential surface. The central axis of the rod wall pipe 3 is the central axis of the puncture core assembly. The above directions of the rod wall pipe 3 are the corresponding directions of the puncture core assembly, and the above surfaces of the rod wall pipe 3 are the corresponding surfaces of the puncture core assembly. The outer surface of the puncture core assembly refers to the outer surface of the rod wall pipe 3.

During an operation, a doctor cuts a small incision in the abdomen of a patient at first, uses the puncturing tip 7 of the puncture core assembly provided by the present disclosure to puncture human tissue to form a puncture opening, and inserts the lower part of the cannula component into the human body, separates the puncture core assembly from a cannula by pressing the insertion block component 8, pulls out the puncture core assembly from the cannula, and then extends a surgical instrument into the cannula to perform an operation. After the operation is completed, the doctor takes out the surgical instrument, inserts the puncture core assembly into the cannula again, clamps the puncture core assembly with the cannula component through the insertion block component 8 again, and starts a suture operation. According to the operation sequence, the suture operation process using the puncture core assembly provided by the present disclosure includes the following actions or steps: positioning, forming a suture channel, extending out a needle and suturing, releasing a suture line, receiving the needle, fixing the needle, and resetting. Further, after the puncture core assembly is reset, the doctor pulls out the puncture core assembly and ties the suture line. The operation of extending out the needle and suturing and the operation of releasing the suture line are started synchronously, and the operation of releasing the suture line is completed before the operation of extending out the needle and suturing.

Referring to Fig. 3, the doctor manipulates the operating component 1 to generate a driving force, and the driving force is transmitted to the execution component 6 and the puncturing tip 7 by the transmission component 4 to enable the execution component 6 and the puncturing tip 7 to perform a suture operation. The operating component 1 includes a pressure cover 12 and a turntable 11 from top to bottom, the transmission component 4 includes a second transmission component 42, a first transmission component 41 and a third transmission component 43 disposed from inside to outside in a sleeving manner, and the execution component 6 includes a positioning component 61, a suture component 62, a receiving component 63 and a needle fixing component 64. Specifically, the turntable 11 is manipulated to rotate, the turntable 11 rotates to drive the first transmission component 41 to rotate, the first transmission component 41 drives the positioning component 61 to rotate, and the positioning component 61 rotates to perform a positioning action and form the suture channel, so that the positioning action is synchronized with the formation of the suture channel. The pressure cover 12 is manipulated to move, the pressure cover 12 moves to drive the second transmission component 42 and the third transmission component 43 to move, the second transmission component 42 moves to synchronously drive the suture component 62 to act and drives the puncturing tip 7 to open, the suture component 62 acts to perform the action of extending out the needle and suturing, and the puncturing tip 7 opens to perform a suture line releasing action, so that the needle extending action and the suture line releasing action are synchronized. The needle receiving component 63 does not need to be driven, and the needle receiving component 63 performs a needle receiving action. The third transmission component 43 moves to drive the needle fixing component 64 to move, and the needle fixing component 64 moves to perform a needle fixing action. Reset refers to the reset of the positioning component 61, the suture component 62 and the puncturing tip 7. A positioning operating component includes a turntable 11, a suture operating component, a suture line releasing operating component or a suture needle fixing operating component all include a pressure cover 12, and the positioning component includes a positioning execution component. The suture component includes a suture execution component, and the suture execution component includes a suture needle. The puncturing tip is called a suture line releasing execution component. The first transmission component is also called a positioning transmission component, the second transmission component is also called a suture transmission component and a suture line releasing transmission component, and the third transmission component is also called a suture needle fixing transmission component.

Referring to Fig. 4 and Fig. 6, the turntable 11 is an integrally formed part including a housing 111 and a shift arm 112. The housing 111 is covered above the butting disc 2 to protect the transmission component 4 above the central through hole 21. The cover body 9 covers the housing 111. The outer end of the shift arm 112 is located outside the cover body 9, the shift arm 112 sequentially passes through a circumferential through hole 92 of the cover body 9 and the housing 111, and the inner end of the shift arm 112 is located inside the housing 111. Referring to Fig. 3, the circumferential through hole 92 provides a space for the shift arm 112 to move circumferentially. Also referring to Fig. 4, the outer end of the shift arm 112 is an operating end, and the inner end of the shift arm 112 is clamped with a first transmission pipe 411 of the first transmission component 41. The first transmission component 41 is sleeved in the rod wall pipe 3. The first transmission component 41 sequentially includes the first transmission pipe 411, two symmetrical transmission arms 412 and a rotating ring 413 from top to bottom. The first transmission pipe 411 is a hollow pipe extending along the axial direction. The near end of the first transmission pipe 411 is located above the central through hole 21 and is clamped with the inner end of the shift arm 112. The far end of the first transmission pipe 411 is fixedly connected with the near end of the transmission arm 412. The two symmetrical transmission arms 412 extend axially, the near end of the transmission arm 412 is fixed to the outside of the first transmission pipe 411, and the far end of the transmission arm 412 is integrally formed with the rotating ring 413. The rotating ring 413 is located between the first supporting piece 51 and the second supporting piece 52, and the rotating ring 413 is drivably connected with the positioning component 61.

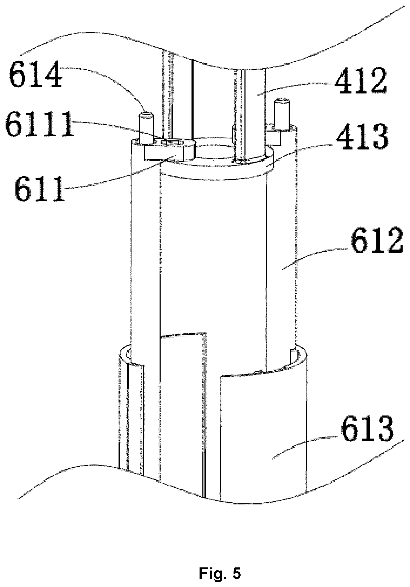

The positioning component 61 includes two symmetrically disposed positioning pieces (not labeled) with the same structure, one is pivotally connected with the first supporting piece 51 and the other is pivotally connected with the second supporting piece 52. The upper end of each positioning piece is provided with a protruding part 614 extending upwardly along the axial direction, the lower end is provided with a protruding part 614 extending downwardly along the axial direction, both the first supporting piece 51 and the second supporting piece 52 are provided with receiving holes 55 extending upwardly and downwardly along the axial direction, each of the protruding parts 614 is received in the corresponding receiving hole 55 and rotates in the receiving hole 55, thus, one positioning piece is pivotally connected with the first supporting piece 51, and the other positioning piece is pivotally connected with the second supporting piece 52. For simplicity of description, only the positioning piece pivotally connected to the first supporting piece 51 is described below. The positioning piece includes a movement aiding arm 611, a pivot shaft 612 and a positioning blade 613. The pivot shaft 612 extends axially, the movement aiding arm 611 is perpendicular to the pivot shaft 612, and the movement aiding arm 611 is drivably connected with the rotating ring 413, so as to realize the rotation of the rotating ring 413 to drive the movement aiding arm 611 to rotate with a connecting line between the upper and lower protruding parts 614 as the axis (i.e. the axis of the pivot shaft 612), and thereby realize the rotation of the rotating ring 413 to drive the positioning piece to rotate with the pivot shaft 612 as the axis. The drivable connection means that the rotation of the rotating ring 413 drives the movement aiding arm 611 to rotate. Specifically, the rotating ring 413 is provided with an upward protruding part (also known as a movement aiding body) (not shown in the figures). As illustrated in Fig. 5, the movement aiding arm 611 is provided with a waist-shaped groove 6111, the movement aiding body is received in the waist-shaped groove 6111, and the movement aiding body moves in the waist-shaped groove 6111. When the rotating ring 413 rotates in the circumferential direction, the movement aiding body also rotates in the circumferential direction. The movement aiding body rotates in the circumferential direction to move in the waist-shaped groove 6111 and drive the waist-shaped groove 6111 to rotate with the pivot shaft 612 as the axis. The movement aiding arm 611 where the waist-shaped groove 6111 is located also rotates with the pivot shaft 612 as the axis, so that the positioning blade 613 rotates outwardly and inwardly with the pivot shaft 612 as the axis. The first supporting piece 51 includes an avoidance space 511, the movement aiding arm 611 is located in the avoidance space 511, and one end of the movement aiding arm 611 is integrally formed with the near end of the pivot shaft 612. The pivot shaft 612 extends axially, and the positioning blade 613 is integrally formed at the far end of the pivot shaft 612. When the positioning blade 613 does not rotate (i.e., in the closed state), the positioning blade 613 is flush with the outer surface of the rod wall pipe 3 and the outer surface of the first supporting piece 51. After the positioning blade 613 rotates (i.e., in the open state), the positioning blade 613 protrudes out of the outer surface of the rod wall pipe 3 and the outer surface of the first supporting piece 51. Therefore, after rotation, the two positioning blades 613 protrude out of the outer surface of the rod wall pipe 3 and butt against tissue on both sides of the puncture opening, so as to realize a positioning function. At this time, the suture component 62 is in a suturable position. Since the upper surface of the positioning blade 613 and the tissue are in surface contact, and the upper surface is perpendicular to the axis of the puncture core assembly, the positioning is more accurate. Since the positioning blade 613 rotates with one axial direction of the puncture core assembly as the central axis, the contact area between the positioning blade 613 and the tissue is not affected by the position of the positioning blade 613 after rotation. Since the upper surface of the positioning blade 613 and the tissue are in surface contact, instead of point contact, the positioning is more accurate.

When the turntable 11 does not rotate but in the initial position, the shift arm 112 is located at one end of the circumferential through hole 92. The doctor shifts the shift arm 112 along the first circumferential direction, the shift arm 112 rotates in the circumferential through hole 92 along the first circumferential direction, the shift arm 112 drives the first transmission component 41 to rotate along the first circumferential direction, the first transmission pipe 411 rotates along the first circumferential direction, the first transmission pipe 411 drives the transmission arm 412 to move along the first circumferential direction, the transmission arm 412 drives the rotating ring 413 to rotate along the first circumferential direction, the rotating ring 413 drives the two symmetrical movement aiding arms 611 of the positioning component 61 to rotate, the two symmetrical movement aiding arms 611 respectively drive the two pivot shafts 612 to rotate, the two pivot shafts 612 respectively drive the two positioning blades 613 to pivot outwardly, so that the two positioning blades 613 change from a state flush with the outer surface of the rod wall pipe 3 to a state protruding out of the outer surface of the rod wall pipe 3, that is, from the closed state to the open state, and the two positioning blades 613 protrude out of the outer surface of the rod wall pipe 3 and are butted against the tissue on both sides of the puncture opening, so as to realize the positioning function. When the shift arm 112 rotates to the other end of the circumferential through hole 92, the hole wall of the circumferential through hole 92 prevents the shift arm 112 from continuing to rotate along the first circumferential direction, so that the positioning blades 613 stop rotating, and at this time, the turntable 11 is in the termination position. When the positioning component 61 performs a reset action, the shift arm 112 is shifted along a second circumferential direction, and the first circumferential direction is opposite to the second circumferential direction. According to the above action transmission relationship, the first transmission component 41 rotates along the second circumferential direction to drive the two pivot shafts 612 to rotate reversely, and the two pivot shafts 612 respectively drive the two positioning blades 613 to pivot inwardly, thus, the two positioning blades 613 are restored from a state protruding out of the outer surface of the rod wall pipe 3 to a state flush with the outer surface of the rod wall pipe 3, that is, from the open state to the closed state, so as to realize the reset of the positioning component 61.

There is a space between the first supporting piece 51 and the second supporting piece 52, which is defined as a receiving space A for receiving the suture component 62. When the positioning blades do not rotate, the positioning blades close the part where the suture component 62 is located in the receiving space A. After the positioning blades rotate, the part where the suture component 62 is located in the receiving space A is exposed to expose a suture channel 53. The suture component 62 moves to the outside of the rod wall pipe 3 through the suture channel 53 to perform a needle extending action.

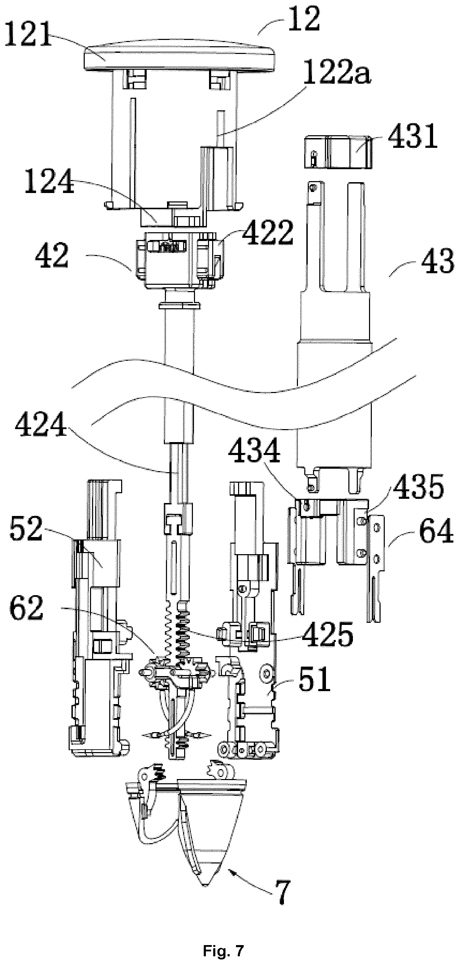

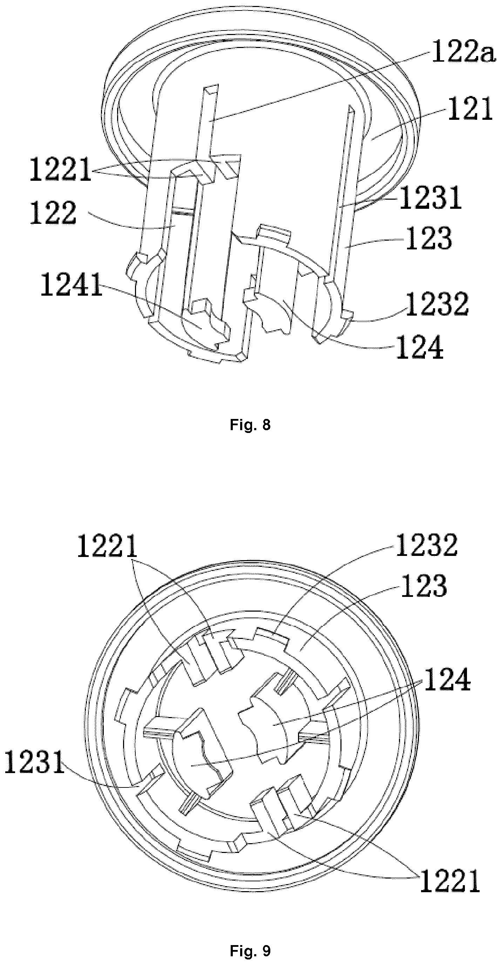

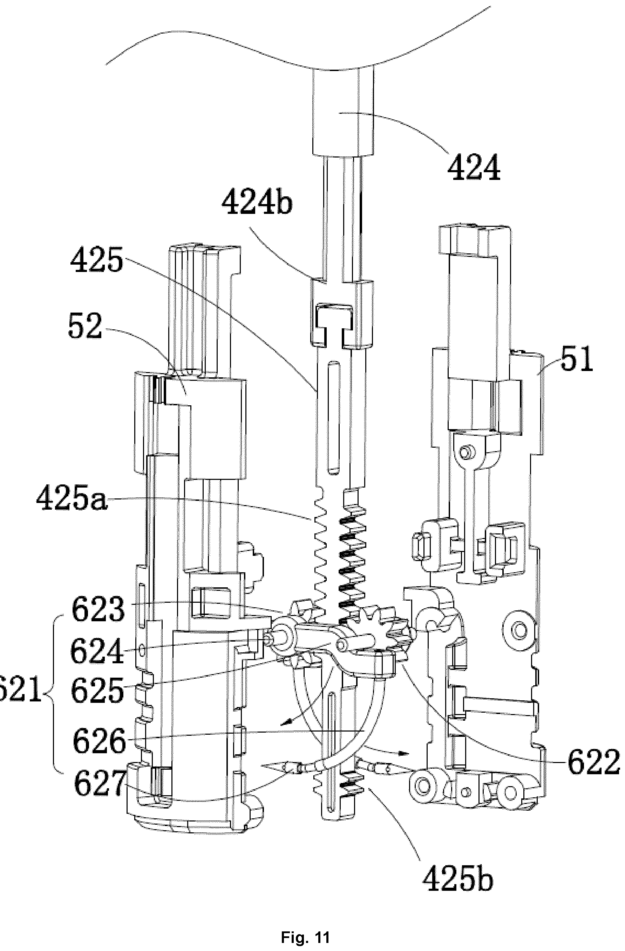

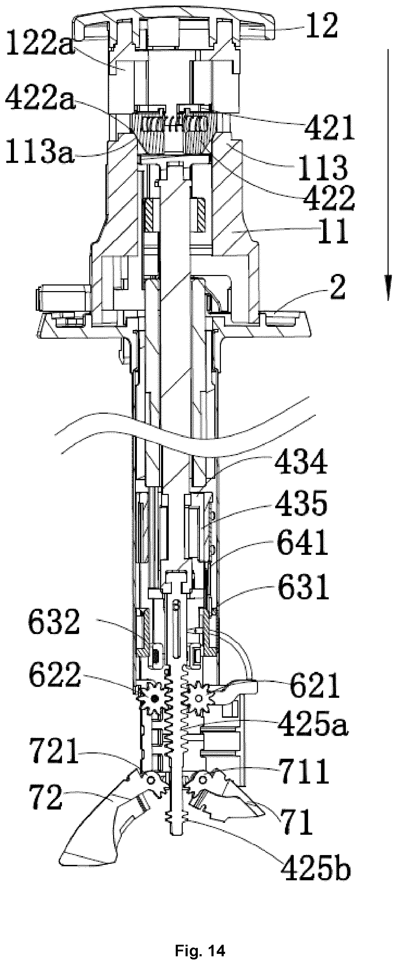

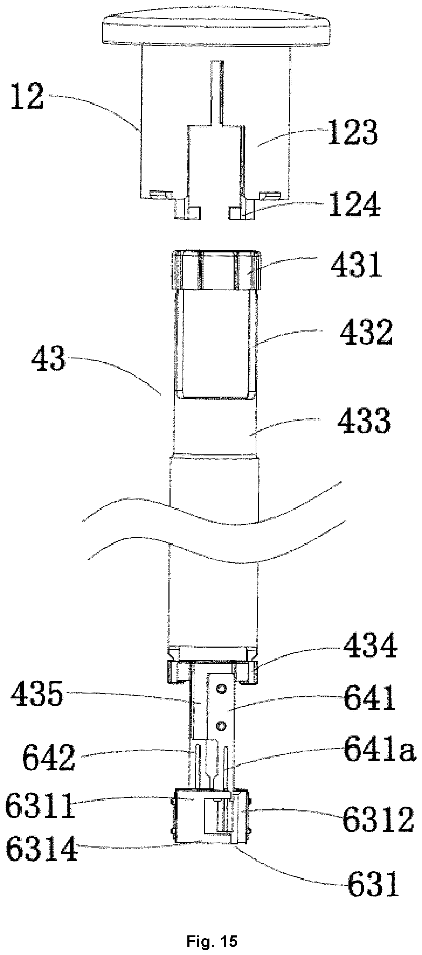

Referring to Figs. 7-9, the pressure cover 12 includes a pressing disc 121, a circumferential wall 123 and two symmetrical pushing members 124, the pressing disc 121 is exposed to the cover body 9, and the near end of the circumferential wall 123 and the near ends of the two pushing members 124 are integrally formed on the lower surface of the pressing disc 121 and extend axially. The circumferential wall is provided with two symmetrical notches 122, both of which are formed after removing a part of the circumferential wall 123. The notches 122 include two slits 122a extending along the axial direction. Both sides of the inner side of each slit 122a protrude inwardly along the radial direction to form two rib plates. The above two rib plates are called a group of rib plates. Each group of rib plates is defined as a rib plate group 1221, and the two rib plate groups 1221 and the two pushing members 124 are disposed in a staggered manner. The axial length of each pushing member 124 is greater than that of the circumferential wall 123, and the far end of the pushing member 124 protrudes inwardly along the radial direction to form a pushing member plate 1241. When the pressure cover 12 is not pressed and is in the first position, each rib plate group 1221 butts against an adjusting block 422 of the second transmission component 42. When the pressure cover 12 is pressed, the rib plate group 1221 presses down the adjusting block 422, so that the second transmission component 42 where the adjusting block 422 is located moves downwardly. As illustrated in Figs. 11-13, the second transmission component 42 moves downwardly to drive the suture component 62 to perform the needle extending action, and the puncturing tip 7 to perform the suture line releasing action. When the action of extending out the needle and suturing is completed, as illustrated in Fig. 14, the pressure cover 12 moves to a second position. The pressure cover 12 is continuously pressed. The two pushing member plates 1241 butts against an upper transmission ring 431 of the third transmission component 43. The pressure cover 12 is continuously pressed, so that the pushing member plate 1241 presses down the upper transmission ring 431, so that the third transmission component 43 where the upper transmission ring 431 is located moves downwardly. As illustrated in Figs. 15-17, the third transmission component 43 moves downwardly to drive the needle fixing component 64 to perform the needle fixing action. After the needle fixing action is completed, the pressure cover 12 moves to a third position. The suture component, the puncturing tip and the needle fixing component share the pressure cover 12 for operation, which reduces the number of operating assemblies and improves the operation convenience.

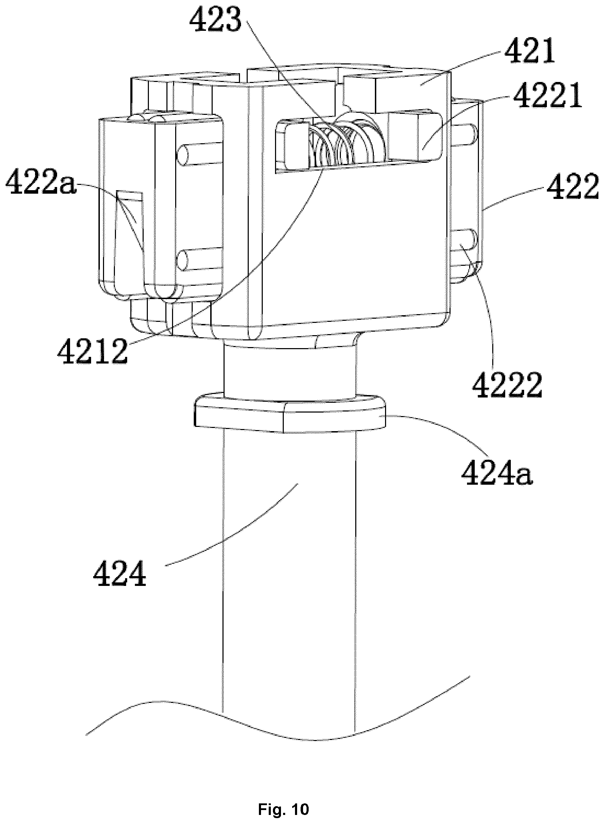

Referring to Fig. 7, the second transmission component 42 includes an adjusting part (not labeled), a transmission rod 424, and a rack 425. Referring to Fig. 10, the adjusting part includes an elastic piece 423, an adjusting frame 421 and two symmetrical adjusting blocks 422. The elastic piece 423 is a spring, the elastic piece 423 is located between the two adjusting blocks 422, each adjusting block 422 is disposed, so that a part of the adjusting block moves radially between the inside and outside of the adjusting frame 421, and the middle part of the adjusting frame 421 is received between the two pushing members 124. When the pressure cover 12 is in the first position and the second position, and in the process of moving from the first position to the second position, due to the elastic force of the elastic piece 423, a certain distance is maintained between the two adjusting blocks 422, a part of the adjusting block 422 is exposed out of the adjusting frame 421, the adjusting part is in the first state, and each rib plate group 1221 butts against the adjusting block 422 on the same side. When the pressure cover 12 moves downwardly from the second position to the third position, the elastic piece 423 shrinks, the adjusting block 422 is received in the adjusting frame 421, and the adjusting part is in the second state. The downward extending cylindrical part of the adjusting frame 421 is fixedly connected with the near end 424a of the transmission rod 424. The near end 424a of the transmission rod 424 is located above the two pushing member plates 1241, and the radial distance between the two pushing member plates 1241 is less than the width of the near end 424a. In this way, when the pressure cover 12 is pulled, the pushing member plates 1241 butt against the near end 424a of the transmission rod 424, thus, the second transmission component 42 where the near end 424a is located is pulled to move upwardly, and the second transmission component 42 moves upwardly to drive the suture component 62 and the puncturing tip 7 to perform the reset action. The transmission rod 424 extends downwardly in the axial direction and passes through the first transmission component 41 to form a far end 424b, which is connected with the rack 425. Teeth are disposed on both sides of the rack 425. The teeth on both sides are divided into two segments from top to bottom, namely, an upper toothed segment 425a and a lower toothed segment 425b. Teeth are symmetrically disposed on both sides of the upper toothed segment 425a. The upper toothed segment 425a transmits a driving force to the suture component 62 to drive the suture component 62 to perform the action of extending out the needle. Teeth are disposed on at least one side of the lower toothed segment 425b, and the lower toothed segment 425b transmits the driving force to the puncturing tip 7 to drive the puncturing tip 7 to perform the suture line releasing action.

Referring to Fig. 11, the suture component 62 includes suture pieces 621 and 622 with the same structure. For simplicity of description, only the suture piece 621 is described below. The suture piece 621 includes a gear 623, a rotating shaft 624, a first suture arm 625, a second suture arm 626, and a suture needle 627. The teeth on one side of the upper toothed segment 425a are cooperated with the gear 623. The gear 623 is sleeved and fixed on the rotating shaft 624. One end of the rotating shaft 624 is rotatably connected with the first supporting piece 51, and the other end is rotatably connected with the second supporting piece 52. The rotating shaft 624 is fixed with one end of the first suture arm 625. The first suture arm 625 is an arm with an avoidance bending part, which is used to give way to the rotating shaft of the suture piece 622, so that the rotating shaft of the suture piece 622 and the rotating shaft of the suture piece 621 are located in the same transverse plane, and the other end of the first suture arm 625 is fixed with one end of the second suture arm 626. The second suture arm 626 is a bent arm, and the other end of the second suture arm 626 is separably connected with the suture needle 627. One end of the suture line is tied to the suture needle 627, and the other end of the suture line is tied to the suture needle of the suture piece 622. When the rack 425 moves downwardly in the axial direction, the teeth on one side of the upper toothed segment 425a drive the gear 623 to rotate, the gear 623 rotates to drive the rotating shaft 624 to rotate in the first direction, and the rotating shaft 624 drives the first suture arm 625, the second suture arm 626 and the suture needle 627 to rotate in the first direction. When the suture needle 627 rotates in the first direction, an end of the suture line is driven to rotate from the inside of the puncture core assembly to the outside of the puncture core assembly and pass through the human tissue on one side of the puncture opening. Teeth on the other side of the upper toothed segment 425a drive the suture piece 622 to rotate in the second direction in the same way, so that the other end of the suture line also rotates from the inside of the puncture core assembly to the outside of the puncture core assembly and passes through the human tissue on the other side of the puncture opening. The first direction is opposite to the second direction.

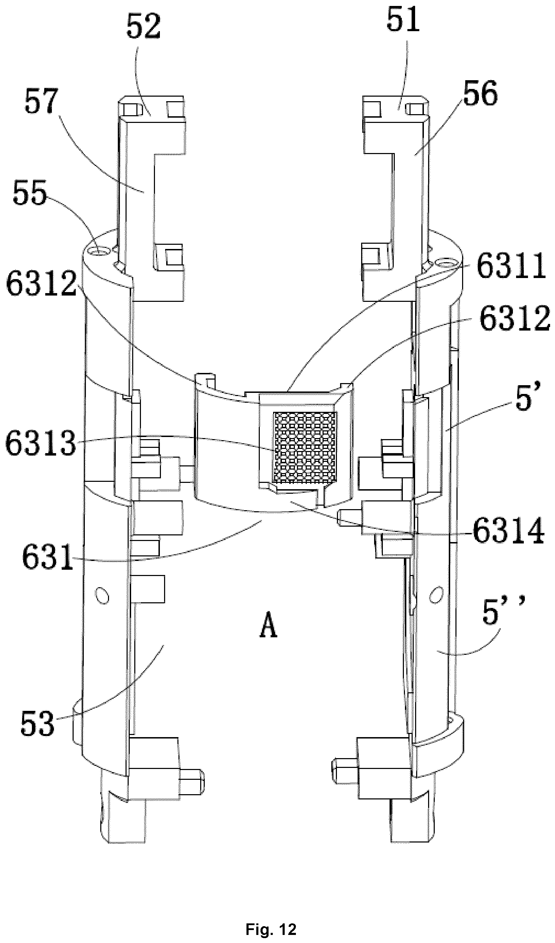

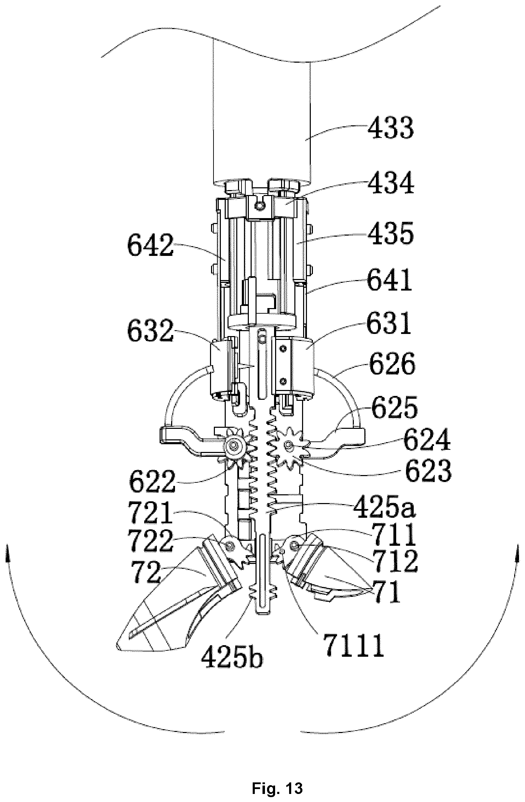

Referring to Fig. 12, after the suture needle drives the end of the suture line to pass through the human tissue around the puncture opening, the suture needle is received by the receiving component 63. The receiving component 63 is disposed on the upper half part 5' of the supporting component 5. The receiving component 63 includes two receiving pieces 631 and 632 with the same structure. For simplicity of description, only the receiving piece 631 is described below. The receiving piece 631 includes a receiving part 6311, a holding part 6312 located on both sides of the receiving part 6311, and a receiving sheet 6313 located in the center of the receiving part 6311. The receiving part 6311 and the holding part 6312 are integrally formed, the holding part 6312 has a bent shape, the supporting component 5 is provided with a receiving space matched with the holding part 6312 in shape, the first supporting piece 51 fixes the holding part 6312 on one side, and the second supporting piece 52 fixes the holding part 6312 on the other side, so that the receiving part 6311 is located above the receiving space A, thus, the receiving part 6311 is located in the rotation path of the suture piece 621. The first supporting piece 51 is connected with the second supporting piece 52 through two receiving pieces 631 and 632 with the same structure. The receiving sheet 6313 is disposed on the receiving part 6311. The receiving sheet 6313 is an elastic grid sheet or an elastic hollow sheet with a hollow structure. The grid of the elastic grid sheet or the hollow structure of the elastic hollow sheet is used to hold the suture needle 627. The rod wall pipe 3 is provided with a window 31, and the receiving sheet 6313 is located in the window 31. After passing through the human tissue on one side of the puncture opening, the suture needle 627 passes through the window 31 and is held by the receiving sheet 6313. The suture needle of the suture piece 622 is synchronously held by the receiving sheet of the receiving piece 632 in the same way, so as to receive the suture needle, as illustrated in Fig. 13.

In the present disclosure, the puncture opening is regarded as a hole, the part above the hole is in vitro, the part under the hole is in vivo, the cavity wall tissue of the human body is around the hole, the side surface of the puncture opening refers to the side wall of the hole, and the human tissue on both sides of the puncture opening refers to the cavity wall tissue of the human body around the hole. According to the present disclosure, the puncture opening is sutured from inside to outside, that is, the suture needle drives the suture line to penetrate in from the lowest layer of tissue on both sides of the puncture opening (i.e. the fascia layer) and out from the side surface of the puncture opening, so that the fascia layer is well sutured. In the puncture core assembly provided by the present disclosure, the two ends of the suture line are respectively tied to the suture needles of the suture pieces 621 and 622. Using the above suture mode, there will be a problem how to send the part of the suture line except the two ends into the body cavity before extending out the needle. In order to simplify the description, the part of the suture line except the two ends is defined as a release part. In order to solve the above problems, the present disclosure makes the release part follow the lower half part of the puncture core assembly to pass through the puncture opening and enter the body cavity, specifically: the release part of the suture line is received in the puncturing tip 7, so that the release part follows the puncturing tip 7 to pass through the puncture opening and enter the body cavity. After the release part follows the puncturing tip 7 to enter the puncture opening, it is necessary to release the suture line during the suture operation to make the suture line have sufficient length. In the present disclosure, the suture line releasing action is executed by the puncturing tip 7.

Referring to Figs. 13 and 14, the puncturing tip 7 is conical. The puncturing tip 7 is provided with a tip inner cavity, which receives the release part of the suture line. When the puncturing tip 7 is opened, the release part of the suture line expands to form a curve segment under the puncturing tip 7 due to gravity and self elasticity to realize the function of releasing the suture line. In order to enable the puncturing tip 7 to be opened, the puncturing tip 7 includes a first wall housing 71 and a second wall housing 72 which may be separated from each other. The first wall housing 71 and the second wall housing 72 form a tip inner cavity after being closed. The first wall housing 71 and the second wall housing 72 may be clamped with each other to close firmly. Specifically, a side surface of one of the first wall housing 71 and the second wall housing 72 is provided with a groove (not shown in the figures), and a side surface of the other is provided with a protrusion 73, which is held in the groove, so that the first wall housing 71 and the second wall housing 72 are clamped. The clamping makes that the first wall housing 71 and the second wall housing 72 are not separated when the puncturing tip performs puncturing, and the movement of the second transmission component 42 along the axial direction releases the clamping between the first wall housing 71 and the second wall housing 72 and separates the first wall housing 71 from the second wall housing 72. The first wall housing 71 and the second wall housing 72 are pivoted respectively to separate them from each other. The pivoting refers to the pivoting of the first wall housing 71 and/or the second wall housing 72 relative to the supporting component 5. In order to realize the pivoting of the first wall housing 71 relative to the supporting component 5, a toothed piece 711 is disposed on the first wall housing 71, the toothed piece 711 is sleeved on a first supporting shaft 712 through a first supporting hole, one end of the first supporting shaft 712 is fixed with the first supporting piece 51, the other end is fixed with the second supporting piece 52, therefore, the first supporting shaft 712 is fixed with the supporting component 5, the first wall housing 71 may pivot around the first supporting shaft 712, the toothed piece 721 is disposed on the second wall housing 72, the toothed piece 722 is sleeved on a second supporting shaft 722 through a second supporting hole, one end of the second supporting shaft 722 is fixed with the first supporting piece 51, the other end is fixed with the second supporting piece 52, therefore, the second supporting shaft 722 is fixed with the supporting component 5, and the second wall housing 72 may pivot around the second supporting shaft 722. In order to drive the first wall housing 71 to pivot around the first supporting shaft 711 and the second wall housing 72 to pivot around the second supporting shaft 722, the toothed pieces 711 and 721 are provided with several teeth, which are selectively engaged with the teeth of the lower toothed segment 425b of the rack 425, so that the relative pivoting between the first wall housing 71 and the second wall housing 72 and the rotation of the suture pieces 621 and 622 are synchronously driven by the rack 425. The relative pivoting includes pivoting of the first wall housing 71 and pivoting of the second wall housing 72, and pivoting of one of the first wall housing 71 and the second wall housing 72 and stationary remaining of the other. When only one of the first wall housing 71 and the second wall housing 72 needs to pivot, teeth are disposed on one side of the lower toothed segment 425b of the rack 425 and no teeth are disposed on the other side. In order to simplify the description, only a condition that both the first wall housing 71 and the second wall housing 72 are pivoted is described below: when the puncturing tip 7 is in the initial state (i.e. not opened), the lower toothed segment 425b of the rack 425 is engaged with the toothed pieces 711 and 721. When the rack 425 moves downwardly, the teeth on one side of the upper toothed segment 425a drive the gear 623 to rotate in the first direction, the gear 623 rotates in the first direction to drive the rotating shaft 624, the first suture arm 625, the second suture arm 626 and the suture needle 627 to rotate in the first direction, and the teeth on one side of the lower toothed segment 425b drive the toothed piece 711 to rotate in the first direction, and the toothed piece 711 drives the first wall housing 71 to pivot in the first direction. At the same time, the teeth on the other side of the upper toothed segment 425a drive the suture piece 622 to rotate in the second direction, the teeth on the other side of the lower toothed segment 425b drive the toothed piece 721 to rotate in the second direction, and the toothed piece 721 drives the second wall housing 72 to pivot in the second direction, so that both the first wall housing 71 and the second wall housing 72 pivot, the puncturing tip 7 is opened, then the suture line is released, and at this time, the puncturing tip 7 is in the termination state. Because the present disclosure adopts a mode of suturing the puncture opening from inside to outside, the motion trajectory of the suture needle of each suture piece is: rotating from the inside of the puncture core assembly to the outside of the puncture core assembly, and passing through the human tissue on one side of the puncture opening. This motion trajectory requires each suture piece to rotate 180 degrees. Therefore, the rack 425 needs to move down enough length to make the suture pieces 621 and 622 rotate 180 degrees. However, considering the maximum pivoting angle of the first wall housing 71 and the second wall housing 72 and the downward movement length of the rack 425, when the first wall housing 71 and/or the second wall housing 72 pivot to the maximum angle, the rack 425 still moves downwardly, and the toothed piece 711 and the toothed piece 721 are no longer engaged with the lower toothed segment 425b, at this time, it is necessary to keep the puncturing tip 7 in the termination state, and wait to be driven by the lower gear segment 425b and reset, in order to achieve the above purpose, a side surface of the toothed piece 711 protrudes along the transverse direction to form a first limiting protruding block 7111, the inner surface of the first supporting piece 51 or the second supporting piece 52 is provided with a first limiting clamping groove (not shown in the figures) for the first limiting protruding block 7111 to slide, the size of a part of the first limiting clamping groove is reduced, so as to be in interference fit with the first limiting protruding block 7111, the part of the first limiting clamping groove corresponds to the position where the first limiting protruding block 7111 is located in the first limiting clamping groove when the first wall housing 71 pivots to the maximum angle, when the first wall housing 71 pivots along the first direction, the first limiting protruding block 7111 rotates along the first direction with the toothed piece 711, in the process of rotation, the first limiting protruding block 7111 enters the first limiting clamping groove and slides in the first limiting clamping groove, when the first wall housing 71 pivots to the maximum angle, the first limiting protruding block 7111 is clamped in the first limiting clamping groove, so that the toothed piece 711 where the first limiting protruding block 7111 is located remains in the position, and the toothed piece 711 remains in the position so that the first wall housing 71 remains open. And/or, an end face of the toothed piece 721 protrudes outwardly along the transverse direction to form a second limiting protruding block (not labeled), the inner surface of the first supporting piece 51 or the second supporting piece 52 is provided with a second limiting clamping groove (not shown in the figure) for the second limiting protruding block to side, the size of a part of the second limiting clamping groove is reduced, so as to be in interference fit with the second limiting protruding block, the part of the second limiting clamping groove corresponds to the position where the second limiting protruding block is located in the second limiting clamping groove when the second wall housing 72 pivots to the maximum angle, when the second wall housing 72 pivots along the second direction, the second limiting protruding block rotates along the second direction with the toothed piece 711, in the process of rotation, the second limiting protruding block enters the second limiting clamping groove and slides in the second limiting clamping groove, when the second wall housing 72 is pivoted to the maximum angle, the second limiting protruding block is clamped in the second limiting clamping groove, so that the toothed piece 721 where the second limiting protruding block is located remains in the position, the toothed piece 721 remains in the position, so that the second wall housing 72 remains open, and the first wall housing 71 and/or the second wall housing 72 remain in the open state, so that the puncturing tip 7 remains in the termination state. After the suture pieces 621 and 622 rotate 180 degrees and the suture needle is received by the receiving component 63 and fixed by the needle fixing component 64, the suture component 62 and the first wall housing 71 and/or the second wall housing 72 may perform a reset action. The pressure cover 12 is pulled upwardly, so that the pushing member plate 1241 butts against the near end 424a of the transmission rod 424, the pushing member plate 1241 pulls the transmission rod 424, so that the second transmission component 42 where the transmission rod 424 is located moves upwardly, which makes the rack 425 move upwardly, the teeth on one side of the upper toothed segment 425a of the rack 425 drive the gear 623 to rotate along the second direction, the gear 623 rotates along the second direction to drive the rotating shaft 624, the first suture arm 625, the second suture arm 626 and the suture needle 627 to rotate along the second direction, when the rack 425 moves upwardly for a certain distance, the teeth on one side of the lower toothed segment 425b encounter the toothed piece 711 and are engaged with the toothed piece 711, so as to drive the toothed piece 711 to rotate along the second direction, and the toothed piece 711 drives the first wall housing 71 to pivot along the second direction. At the same time, the teeth on the other side of the upper toothed segment 425a drive the suture piece 622 to rotate along the first direction, the teeth on the other side of the lower toothed segment 425b drive the toothed piece 721 to rotate along the first direction, and the toothed piece 721 drives the second wall housing 72 to pivot along the first direction, so that the first wall housing 71 and the second wall housing 72 pivot towards each other until the first wall housing 71 and the second wall housing 72 are closed to form the puncturing tip 7. At this time, the puncturing tip 7 returns to the initial state, so that the suture component 62 performs the reset action synchronously with the first wall housing 71 and the second wall housing 72. It is to be noted that, the first wall housing 71 and the second wall housing 72 are two asymmetric structures. In this embodiment, the first wall housing 71 is smaller than the second wall housing 72, so that the puncture force is concentrated on the second wall housing 72, so as to avoid unexpected separation between the first wall housing 71 and the second wall housing 72 driven by the reaction force of human tissue on the puncture force concentration point during puncturing. The suture component 62 and the puncturing tip 7 share a rack to drive, which realizes the synchronous action of the two, meets the action logic relationship between the two, and saves the layout space of the puncture device.

Referring to Figs. 14-15, the third transmission component 43 sequentially includes an upper transmission ring 431, two symmetrical connecting rods 432, a third transmission pipe 433, a lower transmission ring 434 and two symmetrical booster arms 435 from top to bottom. The upper transmission ring 431, the third transmission pipe 433 and the lower transmission ring 434 are coaxial and have the same outer diameter. The upper transmission ring 431 is fixed with the near end of the connecting rod 432, the shift arm 112 passes through a space between the two connecting rods 432 and is clamped with the first transmission pipe 411, and the far end of the connecting rod 432 is fixed with the near end of the third transmission pipe 433. The third transmission pipe 433 extends axially between the rod wall pipe 3 and the first transmission pipe 411. The far end of the third transmission pipe 433 is fixed with the lower transmission ring 434. The lower transmission ring 434 is fixed with the near end of the booster arm 435. The booster arm 435 is located between the first supporting piece 51 and the second supporting piece 52, so that the rotation of the booster arm 435 in the third transmission component 43 is prevented by the first supporting piece 51 and the second supporting piece 52, so that the rotation of the third transmission component 43 is prevented. Further, the outer surface of a first limiting part 56 and/or a second limiting part 57 is provided with a convex point (not shown in the figures), the inner wall of the lower transmission ring 434 is provided with a depression (not shown in the figures), and the convex point is received in the depression, so as to prevent the lower transmission ring 434 from moving downwardly along the axial direction due to gravity, so that the third transmission component 43 where the lower transmission ring 434 is located remains stationary when it is not pressed down by the pressure cover 12.

When the pressure cover 12 is pressed from the second position and moves downwardly to the third position, the pushing member plate 1241 of the pressure cover 12 butts against the upper transmission ring 431 and pushes the third transmission component 43 to move downwardly, so that the lower transmission ring 434 moves against the resistance of the convex point, and the lower transmission ring 434 moves downwardly, so that the booster arm 435 moves downwardly along the axial direction between the first supporting piece 51 and the second supporting piece 52, thus, and the needle fixing component 64 is driven to perform the needle fixing action. The end face where the receiving hole 55 is located is the upper end face of the first supporting piece 51 and the second supporting piece 52.

It is to be noted that, since the pressure cover 12 needs to move downwardly for sufficient displacement to rotate each suture piece 180 degrees, namely the needle fixing action is performed after the suture needle is received by the receiving component 63. The above sufficient displacement is defined as h. When the pressure cover 12 is in the first position, the axial distance between the upper transmission ring 434 of the third transmission component 43 and the pushing member plate 1241 is greater than h, so that, when the pressure cover 12 is pressed and moved downwardly from the first position, each suture piece rotates, and the third transmission component 43 remains fixed until each suture piece has rotated 180 degrees after the pressure cover 12 moves downwardly for displacement h, at this time, the second transmission component 42 is fixed and does not drive the suture component 62 to act, the pushing member plate 1241 moves downwardly to abut against the upper transmission ring 434, so as to drive the needle fixing component 64 to perform the needle fixing action.

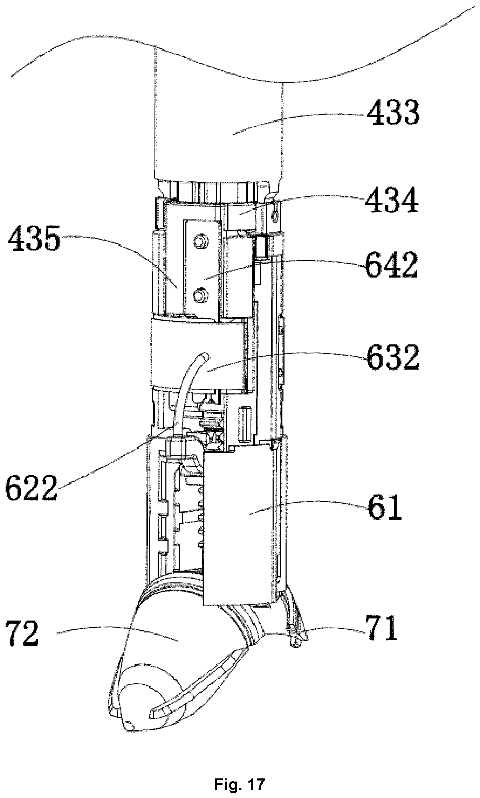

Referring to Figs. 13 and 15, the needle fixing component 64 is disposed above the needle receiving component 63. The needle fixing component 64 includes two symmetrical movable pieces 641 and 642. The movable piece 641 is located above the receiving part 631 in the axial direction, and the movable piece 642 is located above the receiving part 632 in the axial direction. In order to simplify the description, only the movable piece 641 is introduced below. The near end of the movable piece 641 is fixed on the outside of the booster arm 435, and the far end of the movable piece 641 is an open end. The open end is realized by a limiting slit 641a, which is used to further hold the suture needle 627 held on the receiving sheet 6313. When the pressure cover 12 is in the second position, the pressure cover 12 is pressed, the pressure cover 12 moves downwardly to drive the third transmission component 43 to move downwardly, and the third transmission component 43 drives the movable piece 641 to move downwardly. The movable piece 641 moves downwardly to make the suture needle 627 roughly perpendicular to the limiting slit 641a to enter the limiting slit 641a until the suture needle 627 has been clamped into the near end of the limiting slit 641a, the suture needle 627 prevents the near end of the limiting slit 641a from continuously moving downwardly, so that both the movable piece 641 and the third transmission component 43 can not move downwardly, so that the pressure cover 12 can not move downwardly, at this time, the pressure cover 12 is in the third position, as illustrated in Fig. 17. At this time, the far end of the movable piece 641 is radially located between the receiving piece 631 and a blocking arm 6314, and the blocking arm 6314 is formed by extending from the holding part 6312 to the outside of the receiving part 6311. When the pressure cover 12 is in the third position, the pressure cover 12 is pulled, and the pushing member plate 1241 moves upwardly to abut against the near end 424a of the transmission rod 424. The distance that the pushing member plate 1241 moves upwardly to abut against the near end 424a of the transmission rod 424 is equal to the distance that the pressure cover 12 moves from the third position to the second position. The pushing member plate 1241 pulls the transmission rod 424, and then pulls the whole second transmission component 42 to move upwardly. The second transmission component 42 drives the suture component 62 to perform reset. When the suture component 62 performs reset, the rack 425 moves upwardly along the axial direction, the teeth on one side of the upper toothed segment 425a drive the gear 623 to rotate, the gear 623 rotates to drive the rotating shaft 624 to rotate along the second direction, the rotating shaft 624 drives the first suture arm 625 and the second suture arm 626 to rotate along the second direction, the second suture arm 626 supposes to drive the suture needle 627 to move while rotating, however, the movement of the suture needle 627 is prevented by the receiving sheet 6313 and the movable piece 641, in this process, the suture needle 627 gives a reaction force to the receiving sheet 6313 and the movable piece 641, the reaction force causes the movable piece 641 to tilt up, but tilting is blocked by the inner surface of the blocking arm 6314, so that the movable piece 641 maintains the holding of the suture needle 627, therefore, the suture needle 627 is held and separated from the second suture arm 626, after separation, the first suture arm 625 and the second suture arm 626 rotate from the outside of the puncture core assembly and return to the receiving space A, and the suture needle 627 continues to be held in the receiving piece 631, so as to realize the fixation of the suture needle 627. Since an end of the suture line is tied to the suture needle 627, the end of the suture line is also fixed in the receiving piece 631 and the movable piece 641. After the puncture core assembly is pulled out of the body, the end of the suture line is also brought out of the body. By using the same method as described above, the suture needle of the suture piece 622 is fixed by the movable piece 642, so that the other end of the suture line is also fixed in the receiving piece 632 and the movable piece 641, and is also brought out of the body by the puncture core assembly. The movable piece 641 maintains the fixation of the suture needle. The movable piece 641 is cooperated with the blocking arm 6314.

In the present disclosure, erroneous execution of the needle extending action performed by the suture component 62, the suture line releasing action performed by the puncturing tip 7 and the needle fixing action performed by the needle fixing component 64 can be avoided, and the avoidance of erroneous execution is realized by avoiding erroneous driving. Avoidance of erroneous driving of the needle extending action and the suture line releasing action is realized through that the turntable 11 prevents the pressure cover 12 from being wrongly pressed in the first position, and avoidance of erroneous driving of the needle fixing action is realized through that the turntable 11 prevents the pressure cover 12 from being wrongly pressed in the second position.

The fact that the turntable 11 prevents the pressure cover 12 from being wrongly pressed in the first position refers to that: also referring to Figs. 4 and 6, the turntable 11 also includes two symmetrical blocking wings (not labeled). Each blocking wing includes a blocking wing body and a blocking sheet 113. A strip-shaped blocking wing body (not labeled) is formed by protruding outwardly along the radial direction from the outer surface of the upper part of the housing 111, and the top of the blocking wing body extends upwardly along the axial direction beyond the upper end face of the housing 111 to form the blocking sheet 113. As illustrated in Fig. 4, when the turntable 11 is in the initial position, the blocking sheet 113 butts against the circumferential wall 123 of the pressure cover 12, so that the blocking sheet 113 prevents the pressure cover 12 from being wrongly pressed in the first position, so as to avoid the erroneous driving of the needle extending action and the suture line releasing action. As illustrated in Fig. 6, when the turntable 11 rotates from the initial position to the termination position, the turntable 11 rotates along the first circumferential direction until the blocking sheet 113 is staggered with the circumferential wall 123 and aligned with the notch 122. The blocking sheet 113 no longer blocks the downward movement of the pressure cover 12, so that the pressure cover 12 may move downwardly from the first position to the second position, so as to drive the suture component 62 to perform the needle extending action and drive the puncturing tip 7 to perform the suture line releasing action.

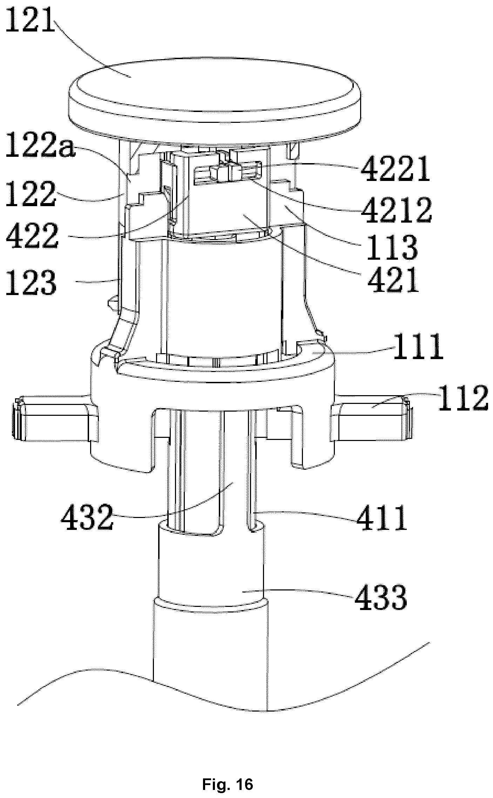

The fact that the turntable 11 prevents the pressure cover 12 from being wrongly pressed in the second position refers to that: also referring to Figs. 14 and 16, the top of each of the two blocking sheets 113 is provided with a first guide inclined plane 113a, the adjusting block 422 is provided with a second guide inclined plane 422a extending between the bottom surface and the side surface, and the first guide inclined plane 113a is cooperated with the second guide inclined plane 422a. As illustrated in Fig. 14, when the pressure cover 12 is in the second position, the first guide inclined plane 113a butts against the second guide inclined plane 422a, the first guide inclined plane 113a prevents the adjustment block 422 from moving downwardly, the adjustment block 422 further prevents the rib plate group 1221 from moving downwardly, and the rib plate group 1221 is prevented from moving downwardly, so that the pressure cover 12 is prevented from being wrongly pressed when in the second position, so as to avoid the erroneous driving of the needle fixing action. A pressing force on the pressure cover 12 is increased, so that the pressure of the rib plate group 1221 on the adjusting block 422 is increased. Since the pressure of the rib plate group 1221 on the adjusting block 422 is increased, the pressure of the second guide inclined plane 422a on the first guide inclined plane 113a is increased. Since the pressure of the second guide inclined plane 422a on the first guide inclined plane 113a is increased, the reaction force of the first guide inclined plane 113a on the second guide inclined plane 422a is increased. The reaction force is decomposed by the second guide inclined plane 422a to form a radial force and an axial force. The radial force is radially inward to make the two adjusting blocks 422 close to each other, so as to shrink the elastic piece 423. The two adjusting blocks 422 close to each other, so that the adjusting block 422 moves from partially exposed to the adjusting frame 421 to fully received in the adjusting frame 421, and the first guide inclined plane 113a no longer butts against the second guide inclined plane 422a, and the adjusting part changes from the first state to the second state. In the second state, the radial length of the adjusting part is reduced, and the adjusting block 422 no longer prevents the rib plate group 1221 from moving downwardly. The preset inclination angles of the first guide inclined plane 113a and the second guide inclined plane 422a makes the adjusting block 422 move against the pressure exerted by the rib plate group 1221. The pressure cover 12 is continuously pressed. Since the radial length of the adjusting frame 421 is less than the radial distance between the two blocking sheets 113, the adjusting frame 421 is received between the two blocking sheets 113. At this time, the two adjusting blocks 422 are subjected to the elastic force of the elastic piece 423 to respectively butt against the inner surface of one of the two blocking sheets 113, so that the adjusting frame 421 is fixed between the two blocking sheets 113. Since the adjusting block no longer prevents the rib plate group 1221 from moving downwardly, when the pressure cover 12 continues to move downwardly, the blocking sheet 113 enters the slit 122a and moves relatively in the slit 122a. In the process, the pressure cover 12 is not in contact with the adjusting part, so that the second transmission component 42 where the adjusting part is located remains fixed without a driving force. At the same time, the pushing member plate 1241 of the pressure cover 12 moves downwardly and touches the upper transmission ring 431, and then pushes the upper transmission ring 431, so as to move the whole third transmission component 43 downwardly, and the third transmission component 43 moves downwardly to drive the needle fixing component 64 to perform the needle fixing action. After the needle fixing action is performed, the pressure cover 12 is located in the third position, as illustrated in Fig. 15.

Preferably, in order to prevent the adjusting block 422 from being completely ejected out of the adjusting frame 421 by the elastic piece 423 when the adjusting block 422 is in the static or moving process, and to avoid the problem of generating severe sloshing due to mutual friction between the adjusting block 422 and the adjusting frame 421 during the movement process of the adjusting block 422, as illustrated in Figs. 10 and 16, the side surface of the adjusting block 422 protrudes outwardly to form a guide protruding block 4221, and the adjusting frame 421 is provided with a guide groove 4212 for the radial movement of the guide protruding block 4221. When the adjusting block 422 is stationary, the guide protruding block 4221 is located in the guide groove 4212, and the guide groove 4212 limits the adjusting block 422 to prevent the adjusting block 422 from being completely ejected out of the adjusting frame 421. When the adjusting block 422 moves, the guide protruding block 4221 moves in the guide groove 4212, and the guide groove 4212 reduces the shaking amplitude when the adjusting block 422 moves. In order to further enhance the movement stability of the adjusting block 422, anti-shaking protruding strips 4222 are disposed on the surface of the adjusting block 422. Due to the anti-shaking protruding strips 4222, the contact area between the adjusting block 422 and the adjusting frame 421 is reduced, thereby reducing the friction between them.

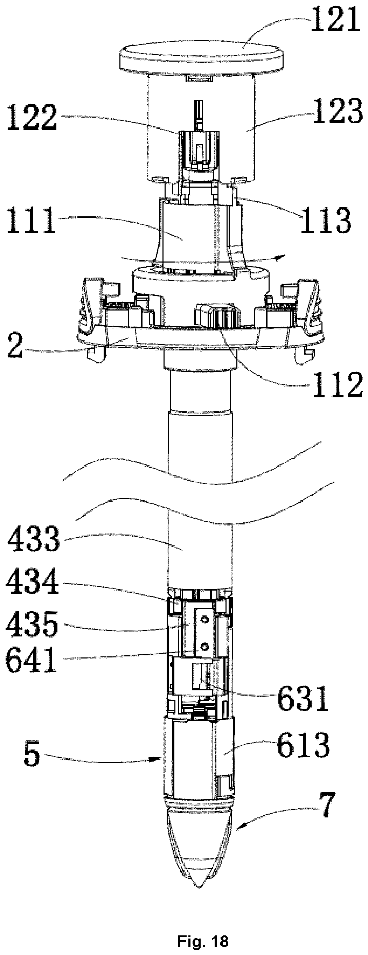

In conclusion, referring to Figs. 2-18, the puncture core assembly provided by the present disclosure is provided with parts for driving, transmitting and executing a suture operation. During suture, a part of the puncture core assembly and a part of the cannula component are located outside the puncture opening, and a part is located in the puncture opening. When the suture operation is started, the shift arm 112 of the turntable 11 is shifted along the first circumferential direction at first, and the shift arm 112 rotates along the first circumferential direction to drive the first transmission component 41 to rotate. The first transmission component 41 drives the positioning blades of the positioning component 61 to rotate outwardly. After the positioning blades pivot outwardly, they protrude out of the outer surface of the rod wall pipe 3, and the puncture core assembly is pulled upwardly, so that the upper ends of the positioning blades butt against the fascia layer on both sides of the puncture opening to realize positioning. After the positioning blades pivoted, the space where the suture component 62 is located in the receiving space A is exposed, which is the suture channel 53. The pressure cover 12 is pressed downwardly, so that the pressure cover 12 moves downwardly. The pressure cover 12 moves downwardly to drive the second transmission component 42 to move downwardly. The upper toothed segment 425a of the rack 425 of the second transmission component 42 moves downwardly to drive the suture component 62 to rotate. The suture piece 621 in the suture component 62 rotates along the first direction, and the suture piece 622 rotates along the second direction to realize extending out the needle and suturing. The two suture needles rotate, and then enter the receiving component 63, the suture needle 627 of the suture piece 621 is received by the receiving piece 631, and the suture needle of the suture piece 622 is received by the receiving piece 632. The lower toothed segment 425b of the rack 425 moves downwardly to drive the relative pivoting of the first wall housing 71 and the second wall housing 72, so as to open the tip inner cavity. The suture line releasing part in the tip inner cavity is separated and expanded from the inner cavity under the action of gravity and the elastic force of the suture line itself to form a curve segment to realize the release of the suture line. The pressure cover 12 is continuously pressed downwardly, so that the pressure cover 12 moves. At this time, the second transmission component 42 and the suture component 62 remain fixed, the pushing member 124 pushes the third transmission component 43 downwardly, the third transmission component 43 pushes the movable pieces 641 and 642 to move downwardly, the limiting slit 641a of the movable piece 641 holds the suture needle 627, and the limiting slit of the movable piece 642 holds the suture needle of the suture piece 622, to achieve needle fixation. The pressure cover 12 is pulled upwardly, the pressure cover 12 moves upwardly to move the second transmission component 42 upwardly, the upper toothed segment 425a of the rack 425 of the second transmission component 42 rotates the suture component 62 in the opposite direction, the two second suture arms of the suture component 62 are separated from the respectively connected suture needles, and the first suture arm and the second suture arm rotate and return to the receiving space A to realize the reset of the suture component 62. The lower toothed segment 425b of the rack 425 enables the first wall housing 71 and the second wall housing 72 to pivot relatively, and the first wall housing 71 and the second wall housing 72 are closed again to realize the reset of the puncturing tip 7. In the process of upwardly pulling the pressure cover 12, the pushing member 124 is far away from the third transmission component 43, and the third transmission component 43 and the needle fixing component 64 remain stationary. After the pressure cover 12 is pulled to the first position, the shift arm 112 is shifted along the second circumferential direction, the shift arm 112 rotates along the second circumferential direction to drive the first transmission component 41 to rotate along the second circumferential direction, and the first transmission component 41 drives the positioning blades to pivot inwardly, and the positioning blades change from the open state to the closed state to realize the reset of the positioning component 61, as illustrated in Fig. 18. Then, the whole puncture device is pulled upwardly, the two ends of the suture line are brought out of the puncture opening by the puncture core assembly, the suture line is cut to separate each end of the suture line from the rest of the suture line, and the suture line is pulled out of the puncture opening to tighten the puncture opening and tie the suture line to complete the suture operation.

The "suturable position" of the present disclosure refers to: when the puncture core assembly is in this position, the suture component 62 is driven, and the suture needle of the suture component 62 may rotate to penetrate in from the tissue around the puncture opening, penetrate out from the side surface of the puncture opening and enter the needle receiving component 63.

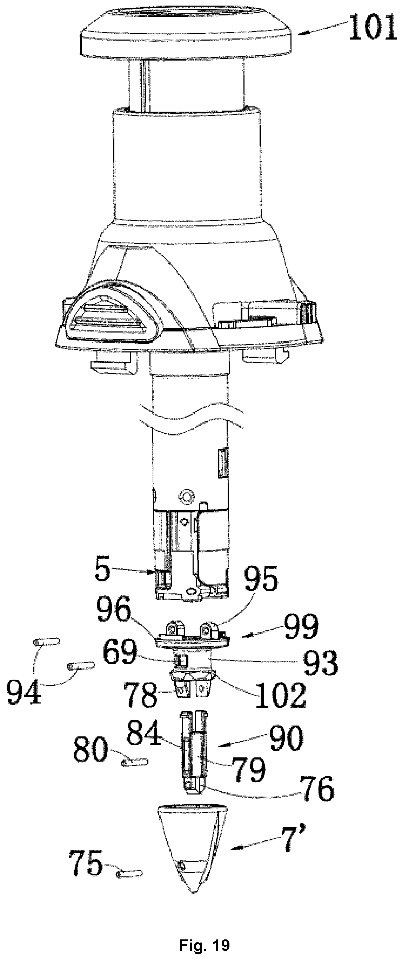

Figs. 19 to 22 illustrate a puncture core assembly of a suturable puncture device according to the second embodiment of the present disclosure.

The following focuses on the difference between the puncture core assembly of the present embodiment and the puncture core assembly of the first embodiment: suture line release mechanisms of the two are different.

In the first embodiment, the suture line release mechanism includes a release execution component, the release execution component includes a puncturing tip, the puncturing tip includes a first wall housing and a second wall housing, the first wall housing and/or the second wall housing pivot relative to a supporting component, so as to switch between an initial state and a termination state, in the initial state, the first wall housing and the second wall housing are closed to form an inner cavity, which is used to receive a suture line, in the termination state, the first wall housing is separated from the second wall housing, and the suture line is released.

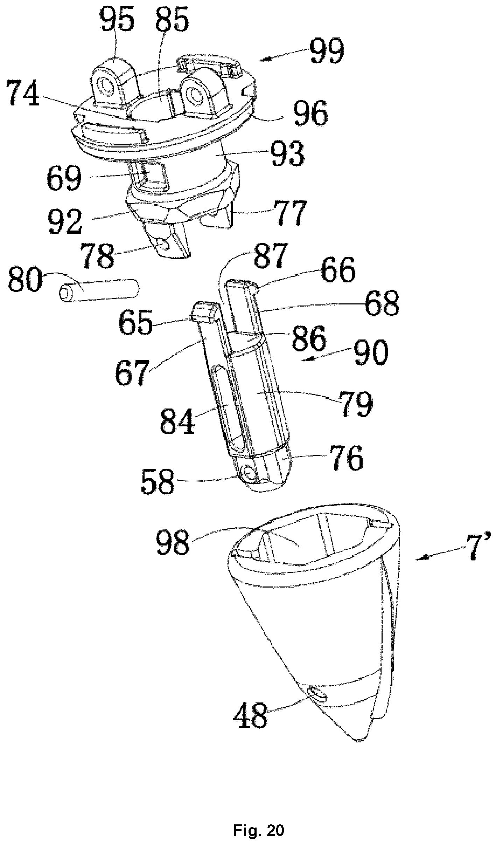

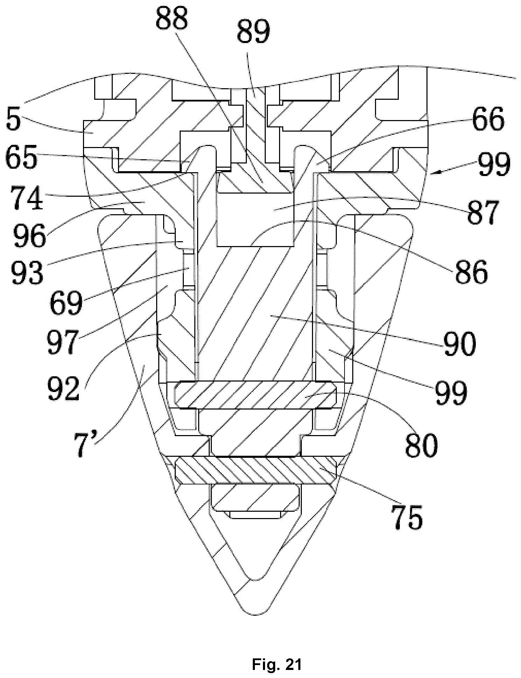

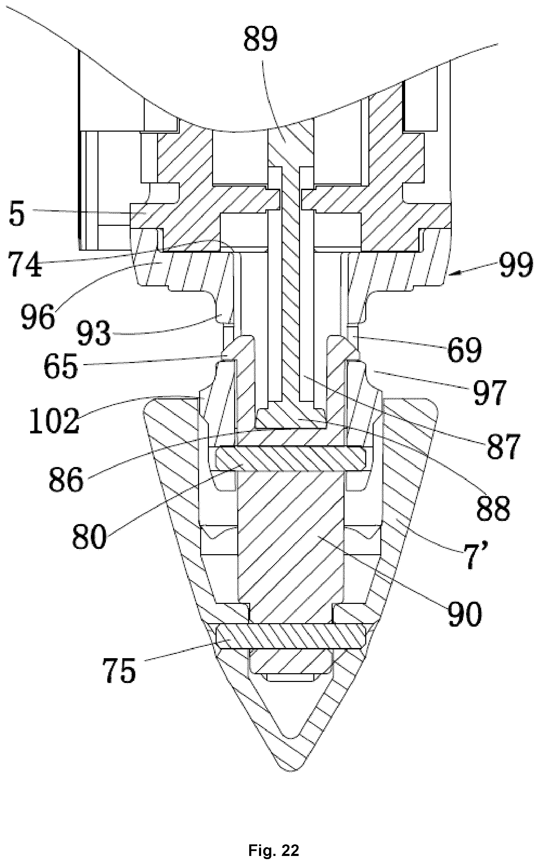

In combination with Fig. 19 and Fig. 20, in this embodiment, a suture line release mechanism includes a release execution component, the release execution component includes a fixing member 99 and a puncturing tip 7', the puncturing tip 7' is provided with a receiving part 98, the fixing member 99 is partially received in the receiving part 98, and the release execution component has an initial state and a release state. In the initial state shown in Fig. 21, a closed suture line receiving space 97 is formed between the puncturing tip 7' and the fixing member 99. For example, in the release state shown in Fig. 22, the suture line receiving space 97 is exposed.

The release execution component is switched from the initial state to the release state. Because the suture line itself has a certain elasticity, when the suture line receiving space 97 is exposed, the suture line will automatically pop up from the suture line receiving space 97 to realize the function of releasing the suture line and prepare for subsequent suture operations. It is to be noted that, the release of the suture line has a process, so that the release state has more than one position. The whole process of the exposure of the suture line receiving space may be regarded as the release state from the exposure of the suture line receiving space 97 to the maximum.

As in the first embodiment, the puncture core assembly of the present embodiment also includes a supporting component. Also referring to Fig. 19 and Fig. 20, in this embodiment, the fixing member 99 is fixed with the supporting component 5. Specifically, the fixing member 99 includes an end plate 96 oppositely located at the near end. The near end face of the end plate 96 is provided with a first mounting part 95, which is fixed with the supporting component 5 through a first fastening pin 94, so that the fixing member 99 is fixed with the supporting component 5.

Referring to Fig. 20 and Fig. 21, the fixing member 99 includes a winding part 93. In the initial state, the suture line is wound around the winding part 93, so that the suture line is regularly received in the suture line receiving space 97. In the initial state, the suture line receiving space 97 is formed between the outer surface of the winding part 93 and the inner surface of the receiving part 98 formed by surrounding the puncturing tip 7'.

The winding part 93 is connected with the far end face of the end plate 96. The axial length of the winding part 93 is greater than the thickness of the end plate 96, so that a sufficient space is provided for suture line winding. The width of the end plate 96 is greater than that of the winding portion 93. In the initial state, the near end face of the puncturing tip 7' butts against the far end face of the end plate 96, so that the suture line receiving space 97 is relatively closed, and the suture line may not be ejected from the suture line receiving space 97.

The fixing member 99 also includes an intercepting plate 102, which is connected with the far end of the winding part 93, and the width of the intercepting plate 102 is greater than that of the winding part 93. Therefore, the axial sections of the end plate 96, the winding part 93 and the intercepting plate 102 are roughly in an "H" shape, so that the suture line is reliably wound on the winding part 93.

The width (i.e., diameter) of the end plate 96 is greater than that of the intercepting plate 102. The wider end plate 96 closes the near end face of the puncturing tip 7' in the initial state, so as to cooperate with the puncturing tip 7' to form the suture line receiving space 97. The narrower intercepting plate 102 reduces the width of the far end of the fixing member 99 on the premise of ensuring the winding effect. Since the part below the end plate 96 of the fixing member 99 is received in the receiving part 98 of the puncturing tip 7', and the width of the intercepting plate 102 in the part below the end plate 96 of the fixing member 99 is the largest, the width of the intercepting plate 102 is reduced to make the size of the fixing member 99 and the size of the puncturing tip 7' smaller, and the size requirements of the puncture core assembly are met.

In the first embodiment, the first wall housing and the second wall housing of the puncturing tip are relatively pivoted to realize the switching between the initial state and the release state.

In this embodiment, the puncturing tip 7' moves along the axial direction of the puncture core assembly, so that the release execution component is switched between the initial state and the release state.

That is, in this embodiment, the puncturing tip 7' no longer includes a first wall housing and a second wall housing which moves relatively. The puncturing tip 7' is formed as a whole, and the puncturing tip 7' moves along the axial direction of the puncture core assembly to realize the switching between the initial state and the release state. Since the puncturing tip 7' is formed as a whole, there is no relative movement between parts, which makes the structure of the puncturing tip 7' more stable, so that the puncturing tip 7' penetrates through tissue more smoothly while puncturing through the tissue. The whole puncturing tip 7' moves along the axial direction to realize the state switching, which is the same as the movement direction of the operating component 101 of the suture line release component, and the structure of the suture line release transmission component is simplified.

In combination with Fig. 19 and Fig. 20, the release execution component includes a lock cylinder 90 fixed with the puncturing tip 7', the suture line release mechanism includes a release transmission component, the release transmission component drives the axial movement of the lock cylinder 90, and the axial movement of the lock cylinder 90 drives the axial movement of the puncturing tip 7'.

The axial movement of the puncturing tip 7' is driven by the lock cylinder 90, which simplifies the structure of the puncturing tip 7'. At the same time, a relevant axial guide mechanism (described subsequently) and a locking mechanism (described subsequently) are disposed on the lock cylinder 90, and the structural design is reasonable.

In combination with Fig. 20 to Fig. 22, the release transmission component includes a transmission rod 89, which butts against the lock cylinder 90, pushes the lock cylinder 90 to move axially, and finally drives the puncturing tip 7' to move axially to switch between the initial state and the release state. The structure is very simple, and the operation is simple and effective.

As in the first embodiment, the suture line release transmission component and the suture transmission component share a transmission rod 89. The transmission rod 89 includes a suture driving part and a suture line release driving part. The suture driving part is a (upper) toothed segment disposed on the side surface of the transmission rod 89. Different from the first embodiment, in the first embodiment, the suture line release driving part is a lower toothed segment, and the lower toothed segment and an upper toothed segment are disposed at intervals. When the lower toothed segment is cooperated with the toothed part disposed on the first wall housing and/or the second wall housing, the lower toothed segment drives the first wall housing and/or the second wall housing to pivot through the toothed part. In this embodiment, the suture line release driving part is a far end part 88 of the transmission rod 89. When the far end part 88 butts against the lock cylinder 90, it pushes the lock cylinder 90 to move axially, so as to drive the puncturing tip 7' to move axially, and finally switch between the initial state and the release state.

In the first embodiment, the lower toothed segment is separably engaged with the toothed part. In this embodiment, the transmission rod separably butts against the lock cylinder 90 along the axial direction. That is, the combination of the transmission rod 89 and the lock cylinder 90 has two states. For example, in the first state shown in Fig. 21, the transmission rod 89 does not push the lock cylinder 90 in the axial direction. For example, in the second state shown in Fig. 22, the transmission rod 89 butts against the lock cylinder 90 in the axial direction and pushes the lock cylinder 90 from the initial state to the release state under driving of an operating part. It is to be noted that, the movement of the transmission rod 89 and the lock cylinder 90 is a process. Therefore, neither the first state nor the second state not just includes a position. Figs. 21 and 22 show one position of the first state and the second state respectively.

Specifically, the lock cylinder 90 is provided with an opening groove 87, which extends from the near end of the lock cylinder 90 towards the far end, the far end of the opening groove 87 is closed to form a far end face 86, and the far end part 88 of the transmission rod 89 butts against the far end face 86 of the opening groove 87 to push the lock cylinder 90 to move axially. Therefore, the opening groove 87 provides guidance for the movement of the transmission rod 89. The width of the far end part 88 of the transmission rod 89 is slightly less than the width of the opening groove 87, so that the far end part 88 slides smoothly in the opening groove 87. The opening groove 87 is formed in the central position of the lock cylinder 90 to make the structure of the lock cylinder 90 regular.

It is to be noted that, whether in the first state or the second state, the far end part 88 of the transmission rod 89 is received in the opening groove 87. The difference is that, for example, in the first state shown in Fig. 21, the far end part 88 of the transmission rod 89 is separated by a certain distance from the far end face 86 of the opening groove 87. For example, in the second state of Fig. 22, the far end part 88 of the transmission rod 89 butts against the far end face 86 of the opening groove 87.

The fixing member 99 is provided with a through hole 85 extending axially, and the lock cylinder 90 is partially received in the through hole 85 and may move in the through hole 85. The through hole 85 provides guidance for the axial movement of the lock cylinder 90, making the movement of the lock cylinder 90 more reliable.

In order to guide the axial movement of the lock cylinder 90 relative to the fixing member 99, an axial guide mechanism is disposed between the lock cylinder 90 and the fixing member 99. The axial guide mechanism includes a guide groove 84 disposed on the lock cylinder 90 and a guide pin 80 disposed on the fixing member 99. The guide groove 84 extends along the axial longitudinal length, and the guide pin 80 is transversely penetrated in the guide groove 84, so that the lock cylinder 90 only moves on the axial path defined by the cooperation of the guide groove 84 and the guide pin 80. The lock cylinder 90 includes a body 79, and the guide groove 84 is disposed in a manner of transversely penetrating through the body 79. The fixing member 99 is provided with a pin hole 78, and the guide pin 80 is closely cooperated with the pin hole 78. The fixing member 99 includes a second mounting part 77, the second mounting part 77 is connected with the intercepting plate 102 and is located at the far end of the fixing member 99 relative to the intercepting plate 102, and the pin hole 78 for mounting the guide pin 80 is disposed on the second mounting part 77. The structural design is reasonable.

A far end part 76 of the lock cylinder 90 located outside the through hole 85 of the fixing member 99 is fixed relative to the puncturing tip 7'. The relative fixation of the lock cylinder 90 and the puncturing tip 7' is realized by the tight fit of a second fastening pin 75, a pin hole 58 disposed on the lock cylinder 90 and a pin hole 48 of the puncturing tip 7', which will not be elaborated here. Therefore, the lock cylinder 90 may drive the puncturing tip 7' to move axially relative to the fixing member 99, so as to switch the suture line execution component between the initial state and the release state.

The release execution component is switched between the initial state and the release state. Accordingly, both ends of the guide groove 84 are closed. When the guide pin 80 butts against the far end of the guide groove 84, the suture line execution component is in the initial state. When the guide pin 80 butts against the near end of the guide groove 84, the suture line execution component is in the final position of the release state. Therefore, both ends of the guide groove 84 limits the axial movement of the lock cylinder 90 and the puncturing tip 7'.

In order to keep the release execution component in the final state of the initial state and the release state, a locking mechanism is disposed between the lock cylinder 90 and the fixing member 99. The locking mechanism includes a hook disposed on the lock cylinder 90, a first holding part 74 and a second holding part 69 disposed on the fixing member 99. The first holding part 74 and the second holding part 69 are disposed at intervals along the axial direction. When the hook is cooperated with the first holding part 74, the release execution component is maintained in the initial state. When the hook is cooperated with the second holding part 69, the release execution component is maintained in the final position of the release state.

Specifically, the lock cylinder 90 includes a body 79 and a first rod part 68 and a second rod part 67 disposed at the near end of the body 79. The first rod part 68 and the second rod part 67 are disposed at intervals, the near end of the first rod part 68 is provided with a first hook 66, and the near end of the second rod part 67 is provided with a second hook 65. The first rod part 68 and the second rod part 67 extend longitudinally and have a certain elasticity. The first rod part 68 and the second rod part 67 are elastically deformed to move the first hook 66 and the second hook 65 respectively, so that the first hook 66 and the second hook 65 switches between the locking state and the release state. The locking state is the state in which the hook is cooperated with the holding part, and the release state is the state in which the hook is separated from the holding part.

The first holding part 74 is disposed on the near end face of the fixing member 99, specifically, on the near end face of the end plate 96. The second holding part 69 is a clamping groove, which penetrates through the side wall of the winding part 93. Compared with the number of hooks, the number of clamping grooves is also two.

As previously mentioned, the fixing member 99 is provided with a through hole 85 extending axially, and the lock cylinder 90 is partially received in the through hole 85 and may move in the through hole 85. A straight line segment formed on the cross section of the first holding part 74 and an arc line segment formed on the cross section of the side wall forming the through hole 85transition smoothly, so as to facilitate the switching of the hook from the locking state to the release state.

As previously mentioned, the lock cylinder 90 is provided with an opening groove 87, and the opening groove 87 provides guidance for the movement of the transmission rod 89. Specifically, the opening groove 87 is formed by being surrounded by the first rod part 68 and the second rod part 67.

Since the positioning mechanism, the suture mechanism and the needle fixing mechanism of the puncture device of this embodiment are the same as those of the first embodiment, the working process of the positioning mechanism, the suture mechanism and the needle fixing mechanism of this embodiment is the same as that of the first embodiment and will not be elaborated. The following focuses on the working process of the suture line release mechanism of the present embodiment.

In the initial state of Fig. 21, both ends of the suture line are connected with the suture needle, the middle part of the suture line is wound on the winding part 93, the hook is cooperated with the first holding part 74, the near end of the puncturing tip 7' butts against the far end face 86 of the end plate 96 of the fixing member 99, and a closed suture line receiving space 97 is formed between the puncturing tip 7' and the fixing member 99, and the suture line wound on the winding part 93 is received in the suture line receiving space 97.

When it is necessary to suture tissue with the puncture core assembly, referring to the changes in Figs. 21 to 22, an operator drives the transmission rod 89 to move down axially by pressing the operating component 101. During the downward movement of the transmission rod 89, the upper toothed segment of the transmission rod 89 drives the suture mechanism to suture the tissue. During the downward movement of the transmission rod 89, the far end part 88 of the transmission rod 89 moves in the direction towards the lock cylinder 90.

Meanwhile, in combination with Fig. 20, when the transmission rod 89 moves down to a position where the far end part 88 butts against the far end face 86 of the opening groove 87 of the lock cylinder 90, the operating component 101 is continuously pressed down, and the transmission rod 89 continues to move down. The driving force provided by the transmission rod 89 causes the elastic deformation of the first rod part 68 and the second rod part 67, so as to move the first hook 66 and the second hook 65, and the first hook 66 and the second hook 65 are separated from the first holding part 74. The transmission rod 89 continues to push the lock cylinder 90 downwardly. The lock cylinder 90 moves downwardly to drive the puncturing tip 7' to move downwardly. The near end face of the puncturing tip 7' gradually moves away from the far end face 86 of the end plate 96. The suture line receiving space 97 begins to be exposed. When the exposed space is large enough, the suture line begins to pop up the suture line receiving space 97 under its own elastic force.

The operating component 101 is continuously pressed down, and the transmission rod 89 continues to move down. During the downward movement, the first hook 66 and the second hook 65 slide in the through hole 85 of the fixing member 99. When the first hook 66 and the second hook 65 are opposite to the second holding part 69, the first hook 66 and the second hook 65 recover from their deformation, and the first hook 66 and the second hook 65 are respectively clamped into the corresponding second holding part 69. When the suture line receiving space reaches the maximum exposure degree and reaches the final position of the release state, the suture line completely released. Of course, the suture line may have been completely released before reaching the final position of the release state, which depends on the elasticity of the suture line.

The embodiments of the present disclosure have been shown or described above. However, it can be understood that the abovementioned embodiments are exemplary and should not be understood as limits to the present disclosure and those of ordinary skill in the art may make variations, modifications, replacements, transformations to the abovementioned embodiments within the scope of the present disclosure.

## Claims

1. A suture line release mechanism for a puncture core assembly, comprising a release execution component, wherein the release execution component comprises a fixing member and a puncturing tip, the puncturing tip is provided with a receiving part, the release execution component has an initial state and a release state, in the initial state, a part of the fixing member is received in the receiving part, a closed suture line receiving space is formed between the puncturing tip and the part of the fixing member, and in the release state, the suture line receiving space is exposed.

2. The suture line release mechanism for the puncture core assembly as claimed in claim 1, wherein the puncturing tip moves along an axial direction of the puncture core assembly, so that the part of the fixing member is received in the receiving part or at least part of the part of the fixing member is removed from the receiving part, so that the release execution component is switched between the initial state and the release state.

3. The suture line release mechanism for the puncture core assembly as claimed in claim 1, wherein the release execution component also comprises a lock cylinder fixed relative to the puncturing tip, the suture line release mechanism also comprises a release transmission component, the release transmission component drives the lock cylinder to move along the axial direction, and an axial movement of the lock cylinder drives the puncturing tip to move along the axial direction.

4. The suture line release mechanism for the puncture core assembly as claimed in claim 3, wherein the lock cylinder comprises a hook, the fixing member comprises a first holding part and a second holding part, the first holding part and the second holding part are disposed at intervals along the axial direction, the hook is cooperated with the first holding part to keep the release execution component in the initial state, and the hook is cooperated with the second holding part to keep the release execution component in the release state.

5. The suture line release mechanism for the puncture core assembly as claimed in claim 3, wherein an axial guide mechanism is disposed between the lock cylinder and the fixing member.

6. The suture line release mechanism for the puncture core assembly as claimed in claim 3, wherein the release transmission component comprises a transmission rod, and the transmission rod butts against the lock cylinder to push the lock cylinder to move axially.

7. The suture line release mechanism for the puncture core assembly as claimed in claim 6, wherein the lock cylinder is provided with an opening groove, the opening groove extends in a direction from an near end of the lock cylinder towards a far end of the lock cylinder, a far end of the opening groove is closed to form a far end face, and a far end part of the transmission rod butts against the far end face of the opening groove to push the lock cylinder to move axially.

8. The suture line release mechanism for the puncture core assembly as claimed in claim 6, wherein the transmission rod separably butts against the lock cylinder along the axial direction.

9. The suture line release mechanism for the puncture core assembly as claimed in claim 3, wherein the fixing member is provided with a through hole that extending axially, and the lock cylinder is movably received in the through hole.

10. The suture line release mechanism for the puncture core assembly as claimed in claim 9, wherein a far end part of the lock cylinder located outside the through hole is fixedly connected with the puncturing tip.

11. A puncture core assembly, comprising the suture line release mechanism as claimed in any one of claims 1 to 10.

12. The puncture core assembly as claimed in claim 11, wherein the suture line release mechanism comprises a release operating component, the puncture core assembly also comprises a suture mechanism, the suture mechanism comprises a suture operating component, and the release operating component and the suture operating component are a same operating component.

13. The puncture core assembly as claimed in claim 12, wherein the puncture core assembly also comprises a suture needle fixing mechanism, the suture needle fixing mechanism comprises a suture needle fixing operating component, and the suture needle fixing operating component, the suture operating component and the release operating component are the same operating component.

14. The puncture core assembly as claimed in claim 11, wherein the same operating component comprises a misoperation prevention mechanism, the misoperation prevention mechanism comprises a first part and a second part, the first part comprises an adjusting block and a spring, one end of the spring butts against the adjusting block, the other end of the spring butts against other adjusting blocks or other parts of the first part except the adjusting block; the second part comprises a blocking part; the blocking part is provided with a first guide inclined plane; the adjusting block is provided with a second guide inclined plane; in an initial position, the first guide inclined plane butts against the second guide inclined plane, when a force of misoperation of the first part is not enough to overcome an elastic force of the spring, the blocking part butts against the adjusting block to prevent the first part from moving relative to the second part due to misoperation; when an operating force is increased, the force overcomes the elastic force of the spring to make the adjusting block move towards other adjusting blocks or the other parts of the first part along the guide inclined plane, and when the adjusting block is separated from the blocking part, the first part is operated to move relative to the second part.

15. The puncture core assembly as claimed in claim 11, wherein the suture line release mechanism comprises a suture line release transmission component, the puncture core assembly also comprises a suture mechanism, the suture mechanism comprises a suture transmission component, the suture line release transmission component shares a transmission rod with the suture transmission component, the transmission rod comprises a suture driving part and a suture line release driving part, the suture driving part is a toothed segment disposed on a side surface of the transmission rod, and the suture line release driving part is a far end part of the transmission rod.

16. A suturable puncture device, comprising the puncture core assembly as claimed in any one of claims 11 to 15 and a cannula component, wherein the puncture core assembly is detachably sleeved in the cannula component.

17. A use method of a suturable puncture device, wherein the suturable puncture device comprises a puncture core assembly and a cannula component, the puncture core assembly is detachably sleeved in the cannula component, the puncture core assembly comprises a first operating component, a second operating component, a third operating component, a positioning blade, a suture piece and a suture line, the suture piece comprises a suture needle, the puncture core assembly comprises a puncturing tip and a fixing member, a suture line receiving space is formed between the puncturing tip and the fixing member, and the use method comprises the following steps:
step S1: operating the first operating component, so as to rotate the positioning blade outwardly, and make the positioning blade protrude out of an outer surface of the puncture core assembly;
step S2: operating the second operating component, so as to rotate the suture piece;
step S3: operating the second operating component, so as to allow the suture line received in the suture line receiving space to be released; and
step S4: operating the third operating component, so as to hold the suture needle.

18. The use method of the suturable puncture device as claimed in claim 17, wherein in the step S1, an operation is rotation.

19. The use method of the suturable puncture device as claimed in claim 17, wherein the step S1 also comprises: exposing a suture channel after the positioning blade rotates outwardly.

20. The use method of the suturable puncture device as claimed in claim 17, wherein in the step S2 and the step S3, an operation is pressing.

21. The use method of the suturable puncture device as claimed in claim 17, wherein the second operating component comprises a pressure cover, the step S3 further comprises: pressing the pressure cover to enable the pressure cover to move downwardly, the pressure cover moves downwardly to drive the puncturing tip to move downwardly, so as to expose the suture line receiving space, and a rotation of the suture needle starts synchronously with an exposure of the suture line receiving space.

22. The use method of the suturable puncture device as claimed in claim 17, further comprising a step S11 between the step S1 and the step S2: upwardly pulling the puncture core assembly or the suturable puncture device, so as to realize a positioning of the positioning blade.

23. The use method of the suturable puncture device as claimed in claim 17, wherein the step S4 further comprises: pressing the third operating component to push a third transmission component, the third transmission component pushes a movable piece to move downwardly, and a slit of the movable piece holds the suture needle.

24. The use method of the suturable puncture device as claimed in claim 17, further comprising the following steps:
step S5: operating the second operating component, so as to make the suture piece return;
step S6: operating the second operating component, so as to close the suture line receiving space;
step S7: operating the first operating component, so as to rotate the positioning blade inwardly; and
step S8: upwardly pulling the puncture core assembly or the suturable puncture device.

25. The use method of the suturable puncture device as claimed in claim 24, wherein in the step S5 and the step S6, an operation is pulling.

26. The use method of the suturable puncture device as claimed in claim 24, wherein in the step S7, an operation is rotation.

27. The use method of the suturable puncture device as claimed in claim 24, wherein the second operating component comprises a pressure cover, the step S6 further comprises: pulling the pressure cover to enable the pressure cover to move upwardly, and the pressure cover moves upwardly to drive the puncturing tip to move upwardly, so as to close the suture line receiving space.
